(19) Europäisches Patentamt
European Patent Office
Office européen des brevets

(11) Publication number: **0 142 333**
**A2**

(12) **EUROPEAN PATENT APPLICATION**

(21) Application number: 84307720.7

(22) Date of filing: 08.11.84

(51) Int. Cl.[4]: C 07 C 93/04
C 07 C 101/18, C 07 C 103/38
A 61 K 31/13, A 61 K 31/14
A 61 K 31/16, A 61 K 31/25

(30) Priority: 08.11.83 JP 208355/83

(43) Date of publication of application: 22.05.85 Bulletin 85/21

(84) Designated Contracting States: AT BE CH DE FR GB IT LI LU NL SE

(71) Applicant: ONO PHARMACEUTICAL CO., LTD.
No. 14, Doshomachi 2-chome Higashi-ku
Osaka-shi Osaka(JP)

(72) Inventor: Arai, Yoshinobu
1-5-8-505, Wakayama-dai
Shimamoto-cho Mishima-gun Osaka(JP)

(72) Inventor: Miyamoto, Tsumoru
1-40 Tonomoriyama
Jyouyou-shi Kyoto(JP)

(72) Inventor: Hamanaka, Nobuyuki
11-38 Hiroshiki
Ohyamazaki-cho Otokuni-gun Kyoto(JP)

(74) Representative: Pearce, Anthony Richmond et al,
Marks & Clerk Alpha Tower Suffolk Street
Queensway Birmingham B1 1TT(GB)

(54) **Glycerol derivatives.**

(57) Glycerol derivatives of the general formula:

1 - $O-R^1$
2 - $A-R^2$ (I)
3 - $B-R^3-R^4$

(wherein $R^1$ represents an alkyl group of 6 to 22 carbon atoms or an alkyl group of 2 to 5 carbon atoms substituted by a phenyl group, A represents a single bond or an oxygene atom, $R^2$ represents an alkyl group of 1 to 20 carbon atoms, an alkenyl group of 2 to 20 carbon atoms when A represents a single bond or an alkenyl group of 3 to 20 carbon atoms when A represents an oxygene atom, B represents an oxygene atom or a carbonyloxy group (i.e. $-O-CO-$ group; with the proviso that in which a carbonyl group is attached to $R^3$ and an oxa-group is attached to a carbon atom at the third position of the glycerol skeleton), $R^3$ represents an alkylene group of 1 to 12 carbon atoms and $R^4$ represents a group of the formula : $-NHCOR^5$, $-N(R^6)_2$ or $-\overset{\oplus}{N}(R^6)_3 \cdot \overset{\ominus}{Y}$ (in which $R^5$ represents an alkyl group of 1 to 6 carbon atoms or a phenyl group, $R^6$ represents an alkyl group of 1 to 6 carbon atoms and $\overset{\ominus}{Y}$ represents an anion of an acid; with the proviso that when plural $R^6$ are attached to a nitrogen atom, they may be same or different to each other)), and acid addition salt thereof, possess antagonistic effect on PAF, hypotensive effect like PAF, inhibitory effect on phospholipase and inhibitory effect on growth of tumour cell or induction of differentiation, and are, therefore, useful as a platelet aggregation inhibitor, anti-asthmatic agent, anti-allergic agent, anti-inflammatory agent, hypotensive agent and anti-tumour agent for mammals.

EP 0 142 333 A2

# D E S C R I P T I O N

## GLYCEROL DERIVATIVES

The present invention is concerned to new glycerol derivatives. More particularly, this invention is concerned to new glycerol derivatives having antagonistic effect on platelet activating factor (abbreviated as PAF hereafter), hypotensive effect like PAF, inhibitory effect on phospholipase and further, inhibitory effect on growth of tumour cell or induction of differentiation, and, furthermore, this invention is concerned to the process for their preparation and pharmaceutical composition comprising them as active ingredients.

Recently, the study on the release reaction of active amine dependent on leukocyte from platelet, has been energetically carried out. As a result, a substance strongly inducing platelet aggregation was discovered and it was named as PAF (cf. J. Immunol., 106, 1244 (1971), J. Exp. Med., 136, 1356 (1972) and Nature, 249, 581 (1974)). Thereafter, it was confirmed that PAF exists in various animals including human beings. In 1979, its chemical structure was identified (cf. J. Biol. Chem., 254, 9355 (1979) and C.R. Acad. Sci., Série D (Paris), 289, 1017 (1979)) and has turned out to be a mixture of two alkylphospholipids of the following general formula wherein n is 15 and 17 (cf. J. Biol. Chem., 255, 5514 (1980)) :

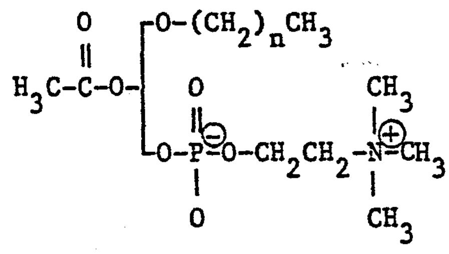

(wherein n represents 15 or 17).

It was found that PAF has not only strong secretory effect on platelet aggregation known at that time, but also powerful hypotensive effect and bronchocontracting effect. PAF is considered to be one of platelet aggregating factors in human beings, and further, one of substances inducing allergy and inflammation. Accordingly, there is a great possibility that compounds having antagonistic effect on PAF (inhibitory effect on PAF) may become a platelet aggregation inhibitor, anti-asthmatic agent, anti-allergic agent or anti-inflammatory agent. Furthermore, PAF is considered to become a hypotensive agent without inhibitory effect on platelet aggregation by selecting hypotensive effect out of various effect.

Being interested in physiological effect on PAF, we have carried out widespread investigations in order to discover compounds similar to PAF in their chemical structure and possessing antagonistic effect on PAF or hypotensive effect like PAF. We have found that the above purpose can be accomplished by glycerol derivatives which are replaced the hydroxy group at the first position of the glycerol

skeleton by an alkoxy group and are replaced the hydroxy group at the third position thereof by an alkoxy group substituted by a primary, secondary, tertiary or quaternary amine, preferably tertiary or quaternary amine.

For example, our European Patent Publication No. 94586 discloses the compounds of the general formula:

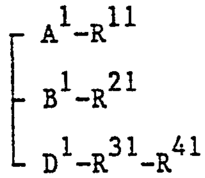

(wherein $A^1$ is -O- or -S-, $B^1$ is -O- or -S-, $D^1$ is -O- or -OCO-, $R^{11}$ is alkyl, $R^{21}$ is H, benzyl, acyl, N-substituted amido when $B^1$ is -O-, or $R^{21}$ is alkyl, benzyl or alkoxy (thiocarbonyl) when $B^1$ is -S-, $R^{31}$ is alkylene and $R^{41}$ is amino, mono- or dialkyl-substituted amino or trialkyl-substituted ammonium) and our European Patent Publication No. 109255 disclose the compounds of the general formula:

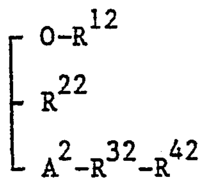

(wherein $A^2$ is -O- or -OCO-, $R^{12}$ is alkyl, $R^{22}$ is azido, amino, acylamino, alkoxycarbonylamino or ureido, $R^{32}$ is alkylene and $R^{42}$ is amino, mono- or dialkyl-substituted amino or trialkyl-substituted ammonium).

Now, we have found that by replacing functioned groups at the second position of the glycerol skeleton, hereinbefore described by an alkyl(oxy) or alkenyl(oxy) group and by limiting optionally

substituted amino group attached to the end of the third position thereof to tertiary or quaternary amine, or amide, new glycerol compounds are more active in antagonistic effect on PAF than glycerol compounds previously proposed, having completed this invention.

On the other hand, glycerol compounds similar to those of the present invention in chemical structure are disclosed in the German Patent Publication No. 2835369. Broad scope of compound is disclosed therein and the compounds of the general formula:

$$\begin{array}{l} CH_2-O-R_{23} \\ | \\ CH-O-R_{13} \\ | \\ CH_2-\!\!\left[O-Y\right]_m\!\!-\!\!\left[NH-Z\right]_n\!\!-\!(CH_2)_pNHR_{33} \end{array} \qquad (A)$$

(wherein $R_{13}$ and $R_{23}$ each represents a normal alkyl or alkenyl group of 12 to 20 carbon atoms and Y and Z each represents an alkylene group of 2 to 4 carbon atoms or ..., $R_{33}$ represents a hydrogen atom, an alkyl group of 2 to 4 carbon atoms or ..., m, n and p each represents zero or 1 and the total number of m, n and p is zero or 1) are most related to our invention. (the definition not related to our invention is removed from the above description.)

As understood from the above definition, $-NHR_3$ in formula (A) in DE-2835369 represents only a primary or secondary amine, which $R^4$ in the general formula (I) hereinafter described, of the present invention represents a tertiary or quaternary amine.

Furthermore, the distinguished difference between the present invention and the invention disclosed in DE-2835369 is utility of glycerol compounds. That is, compounds of the present invention have antagonistic effect on PAF and hypotensive effect like PAF and,

therefore, are used as platelet aggregation inhibitor, anti-asthmatic agent, anti-allergic agent and anti-inflammatory agent, whereas compounds in DE-2835369 have stimulating activity of production of interferon and are useful for prophylaxis of viral disease.

As discussed above, the present invention is remarkably different from the invention disclosed in DE-2835369 in chemical structure and their utility. The present invention is not anticipated and not obvious from DE-2835369.

Furthermore, it has been found that the compounds of the present invention of the general formula (I) have inhibitory effect on phospholipase $A_2$ and phospholipase C. Phospholipase $A_2$ and phospholipase C dependent on calcium in a living body are concerned in the mechanism for the release of arachidonic acid from phospholipids. It is known that arachidonic acid is released by physiological stimulus and then becomes incorporated into the metabolic routes of prostaglandins and is finally metabolized to thromboxane $A_2$ having a strong effect on platelet aggregation, to other prostaglandins or to various leukotrienes being a significant bronchostrictor in an asthmatic paroxysm. Accordingly, it is considered that compounds having an inhibitory effect on phospholipase $A_2$ and phospholipase C, which are concerned in the mechanism for the release of arachidonic acid, can become a platelet aggregation inhibitor, anti-asthmatic agent, anti-allergic agent or anti-inflammatory agent, based on the inhibition of phospholipase $A_2$ and phospholipase C, having a different mechanism from PAF antagonist and further, being different from conventional non-steroid anti-inflammatory agents represented by aspirin based on the inhibition of cyclooxygenase.

Furthermore, the compounds of the present invention of the general formula (I) show inhibitory effect on growth of tumour cell and induction of differentiation and, therefore, are may be useful as anti-tumour agents.

The present invention is concerned to new glycerol derivatives of the general formula:

$$\begin{array}{l} 1 \ \ O-R^1 \\ 2 \ \ A-R^2 \\ 3 \ \ B-R^3-R^4 \end{array} \qquad (I)$$

[wherein $R^1$ represents an alkyl group of 6 to 22 carbon atoms or an alkyl group of 2 to 5 carbon atoms substituted by a phenyl group, A represents a single bond or an oxygen atom, $R^2$ represents an alkyl group of 1 to 20 carbon atoms, an alkenyl group of 2 to 20 carbon atoms when A represents a single bond or an alkenyl group of 3 to 20 carbon atoms when A represents an oxygen atom, B represents an oxygen atom or a carbonyloxy group (i.e. -O-CO- group ; with the proviso that in which a carbonyl group is attached to $R^3$ and an oxa-group is attached to a carbon atom at the third position of the glycerol skeleton), $R^3$ represents an alkylene group of 1 to 12 carbon atoms and $R^4$ represents a group of the formula : $-NHCOR^5$, $-N(R^6)_2$ or $-\overset{\oplus}{N}(R^6)_3 \cdot \overset{\ominus}{Y}$ (in which $R^5$ represents an alkyl group of 1 to 6 carbon atoms or a phenyl group, $R^6$ represents an alkyl group of 1 to 6 carbon atoms and $\overset{\ominus}{Y}$ represents an anion of an acid; with the proviso that when plural $R^6$ are attached to a nitrogen atom, they may be same or different to each other)], and acid addition salt thereof.

The expression of an alkyl group or an alkylene group in the definition of the various symbols on this present specification including claims represents a straight- or branched -chain alkyl or alkylen group.

Examples of an alkyl group of 6 to 22 carbon atoms represented by $R^1$ in the general formula (I) are hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl and docosyl group and isomers thereof; an alkyl group of 14 to 18 carbon atoms is preferred and hexadecyl group is most preferred.

Examples of an alkyl group of 2 to 5 carbon atoms substituted by a phenyl group, represented by $R^1$ are ethyl, propyl, butyl and pentyl group and isomers thereof substituted any one of hydrogen atoms thereof by a phenyl group.

In $R^2$ of the general formula (I), examples of the alkyl group of 1 to 20 carbon atoms are methyl, ethyl, propyl, butyl, pentyl, hexyl, heptyl, octyl, nonyl, decyl, undercyl, dodecyl, tridecyl, tetradecyl, pentadecyl, hexadecyl, heptadecyl, octadecyl, nonadecyl and eicosyl group and isomers thereof, examples of an alkenyl group of 2 to 20 carbon atoms when A represents a single bond, are the groups which have a double bond between two carbon atoms in the alkyl group enumerated above (except methyl group) and examples of an alkenyl group of 3 to 20 carbon atoms when A represents an oxygen atom, are the groups which have a double bond between two carbon atoms except the carbon atom attached to an oxygen atom represented by A in the alkyl group enumerated above (except methyl and ethyl group); the

alkyl group of 1 to 6 carbon atoms is preferred and allyl group is most preferred in the alkenyl group.

Examples of the alkylene group of 1 to 12 carbon atoms represented by $R^3$ in the general formula (I) are methylene, ethylene, trimethylene, tetramethylene, pentamethylene, hexamethylene, heptamethylene, octamethylene, nonamethylene, decamethylene, undecamethylene and dodecamethylene group and isomers thereof; the alkylene group of 3 to 8 carbon atoms is preferred and heptamethylene group is most preferred.

In $R^4$ of the general formula (I), examples of the alkyl group of 1 to 6 carbon atoms represented by $R^5$ or $R^6$ are methyl, ethyl, propyl, butyl, pentyl and hexyl group and isomers thereof; every group is preferred. The anion of an acid represented by $\overset{\ominus}{Y}$ mean the anion of a pharmaceutically-acceptable inorganic or organic acid, and examples of them are the anion of an inorganic acid such as hydrochloric acid, hydrobromic acid, hydroiodic acid, sulfuric acid, phosphoric acid, nitric acid, or the anion of an organic acid such as acetic acid, lactic acid, tartaric acid, benzoic acid, citric acid, methanesulfonic acid, ethanesulfonic acid, benzenesulfonic acid, toluenensulfonic acid, isethionic acid; more prefereable anion is a halogen ion, i.e. chlorine, bromine, iodine ions, or methanesulfonic acid or p-toluenesulfonic acid.

Preferred $R^4$ is the group of the formula: $-N(R^6)_2$ or $-\overset{\oplus}{N}(R^6)_3 \cdot \overset{\ominus}{Y}$ (in which all the symbols are as hereinbefore defined), and the most preferred $R^4$ is the group of the formula : $-\overset{\oplus}{N}(R^6)_3 \cdot \overset{\ominus}{Y}$.

The compounds of the general formula (I) of the present invention have at least one asymmetric center, i.e. the carbon atom at the second position of the glycerol skeleton. When the alkyl moiety of various substituents represented by $R^1$, $R^2$, $R^3$ and $R^4$ represents a branched-chain, there is a possibility that other asymmetric centers are occurred. However the compounds of the general formula (I) in the present invention are concerned with all isomers or mixtures thereof which result from asymmetric centers.

According to a feature of the present invention, compounds of the general formula (I), wherein B represents an oxygen atom and $R^4$ represents a group of the formula: $-N(R^6)_2$ or $-\overset{\oplus}{N}(R^6)_3 \cdot \overset{\ominus}{Y}{}^2$ (in which $Y^2$ represents a halogen atom, alkylsulfonyloxy group or arylsulfonyloxy group and the other symbols are as hereinbefore defined), i.e. compounds of the general formulae:

$$\left[\begin{array}{l} O-R^1 \\ A-R^2 \\ O-R^3-N(R^6)_2 \end{array}\right. \qquad \text{(Ia)}$$

and

$$\left[\begin{array}{l} O-R^1 \\ A-R^2 \\ O-R^3-\overset{\oplus}{N}(R^6)_3 \cdot \overset{\ominus}{Y}{}^2 \end{array}\right. \qquad \text{(Ib)}$$

(wherein the various symbols are as hereinbefore defined) may be

prepared by the reaction of a compound of the general formula:

$$\left[\begin{array}{l} O\text{-}R^1 \\ A\text{-}R^2 \\ O\text{-}R^3\text{-}OR^7 \end{array}\right. \qquad \text{(IIa)}$$

(wherein $R^7$ represents an alkylsulfornyl group, a methanesulfonyl group (i.e. a mesyl group) is preferred or a substituted or unsubstituted arylsulfonyl group, a p-toluenesulfonyl group (i.e. tosyl group) is preferred and the other symbols are as hereinbefore defined)

or a compound of the general formula:

$$\left[\begin{array}{l} O\text{-}R^1 \\ A\text{-}R^2 \\ O\text{-}R^3\text{-}Y^1 \end{array}\right. \qquad \text{(IIIa)}$$

(wherein $Y^1$ represents a halogen atom and the other symbols are as hereinbefore defined), respectively, with a compound of the general formula:

$$NH(R^6)_2 \text{ or } N(R^6)_3$$

(wherein $R^6$ is as hereinbefore defined) in a suitable organic solvent, e.g. a lower alkanol such as methanol, ethanol or isopropylalcohol, or dimethylformamide or the mixture thereof, at a temperture from ambient to a reflux temperature of the solvent.

The compounds of the general formula (I), wherein B represents a carbonyloxy group and $R^4$ represents as a group of the formula : $-N(R^6)_2$ (in which $R^6$ is as hereinbefore defined), i.e.

compound of the general formula:

$$\left[\begin{array}{l} O\text{-}R^1 \\ A\text{-}R^2 \\ O\text{-}CO\text{-}R^3\text{-}N(R^6)_2 \end{array}\right. \qquad (Ic)$$

(wherein all the symbols are as hereinbefore defined) may be prepared by esterification of a compound of the general formula:

$$\left[\begin{array}{l} O\text{-}R^1 \\ A\text{-}R^2 \\ OH \end{array}\right. \qquad (IV)$$

(wherein all the symbols are as hereinbefore defined) with a compound of the general formula:

$$HOOC\text{-}R^3\text{-}(R^6)_2 \qquad (IV\text{-}1)$$

(wherein all the symbols are as hereinbefore defined).

The esterification may be carried out in an inert organic solvent such as ethyl acetate, benzene, ethyl ether, chloroform, methylene chloride or tetrahydrofuran using dicyclohexylcarbodiimide, trifluoroacetic acid anhydride or N,N'-carbodiimidazole as a condensing agent at a temperature from 0°C to 50°C (preferably at ambient). When dicyclohexyl- carbodiimide is used as a condensing agent, 4-dimethylaminopyridine or pyridine etc. is used as a agent of promoting reaction.

The compound of the general formula (I), wherein B represents a carbonyloxy group and $R^4$ represents a group of the formula : $-\overset{\oplus}{N}(R^6)_3 \cdot \overset{\ominus}{Y}^2$ (in which all the symbols are as hereinbefore defined), i.e. compound of the general formula:

$$\left[\begin{array}{l} O\text{-}R^1 \\ A\text{-}R^2 \\ O\text{-}CO\text{-}R^3\text{-}\overset{\oplus}{N}(R^6)_3\cdot\overset{\ominus}{Y}{}^2 \end{array}\right. \qquad \text{(Id)}$$

(wherein all the symbols are as hereinbefore defined) may be prepared by the reaction of a compound of the general formula:

$$\left[\begin{array}{l} O\text{-}R^1 \\ A\text{-}R^2 \\ O\text{-}CO\text{-}R^3\text{-}OR^7 \end{array}\right. \qquad \text{(IIb)}$$

or

$$\left[\begin{array}{l} O\text{-}R^1 \\ A\text{-}R^2 \\ O\text{-}CO\text{-}R^3\text{-}Y^1 \end{array}\right. \qquad \text{(IIIb)}$$

(wherein all the symbols are as hereinbefore defined) with a compound of the general formula : $N(R^6)_3$ (wherein $R^6$ is as hereinbefore defined). The reaction may be carried out under the same condition as mentioned above for the reaction of a compound of the general formula (IIa) or (IIIa) with a compound of the general formula : $N(R^6)_3$.

The compound in which $R^4$ represents a group of the formula : $-NHCOR^5$ (in which $R^5$ is as hereinbefore defined), i.e. compound of the general formula :

$$\left[\begin{array}{l} O\text{-}R^1 \\ A\text{-}R^2 \\ B\text{-}R^3\text{-}NHCOR^5 \end{array}\right. \qquad \text{(Ie)}$$

(wherein all the symbols are as hereinbefore defined) may be prepared by acylation of a compound of the general formula (I) wherein $R^4$ represents an amino group, i.e. compound of the general formula:

$$\left[\begin{array}{l} O-R^1 \\ A-R^2 \\ B-R^3-NH_2 \end{array}\right. \qquad (Ie')$$

(wherein all the symbols are as hereinbefore defined) by a method known _per se_. Suitable acylation may be carried out by using a corresponding acyl chloride, benzoyl chloride or acid anhydride in the presence of a tertiary amine such as pyridine or triethylamine in an inert organic solvent such as methylene chloride or in the absence of a solvent at a temperature below 50°C.

The compounds of the general formulae (IIa), (IIIa), (IIb), (IIIb) and (IV) may be prepared by the series of reactions depicted schematically below in Scheme I. In Scheme I, THP represents a tetrahydropyran-2-yl group, and the other symbols are as hereinbefore defined.

Scheme I

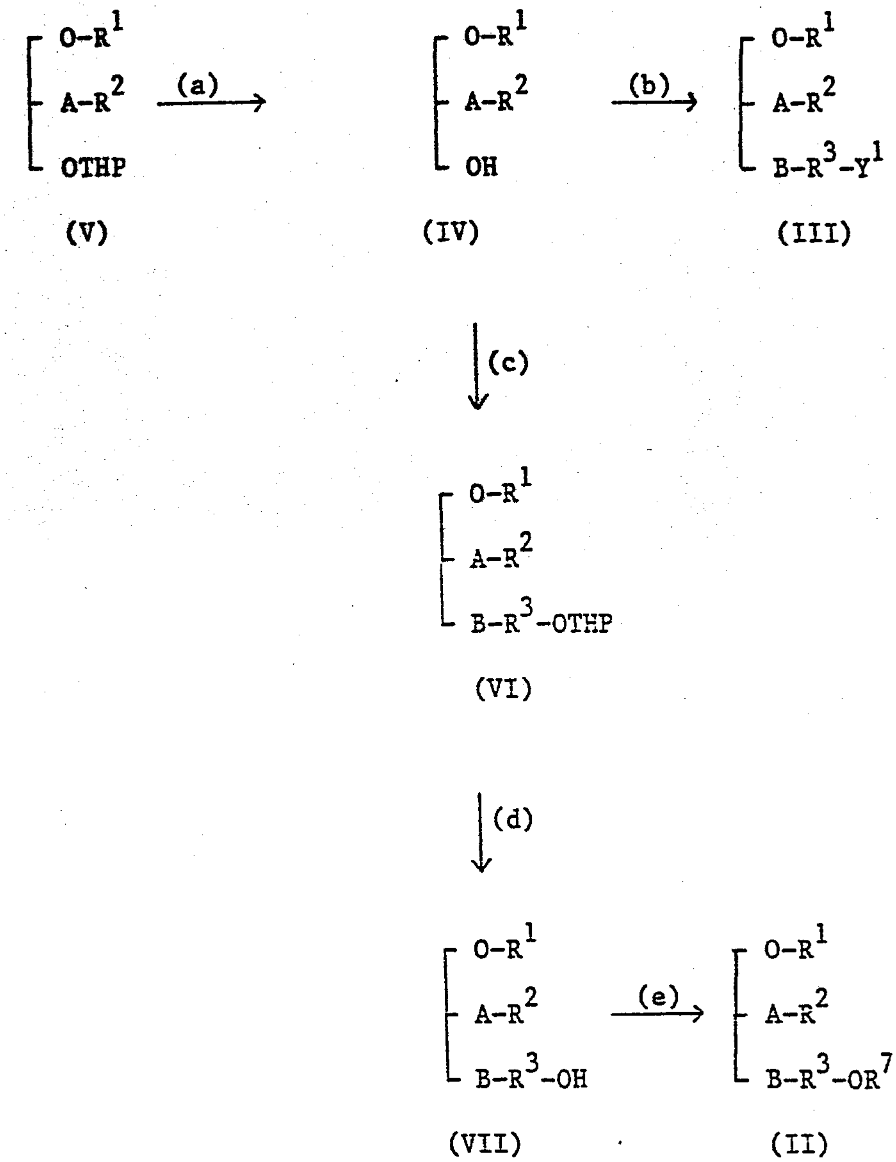


(V) (IV) (III) (VI) (VII) (II)

In Scheme I, each step can be effected by using methods known *per se*. For example, step (a) and (d) are a reaction for eliminating THP group, and may be carried out by using a mixture of tetrahydrofuran, acetic acid and water, p-toluenesulfonic acid and anhydrous methanol or dilute hydrochloride and tetrahydrofuran at ambient or by heating.

Step (b) may be carried out by reacting a compound of the general formula (IV) wherein B represents an oxygen atom, with a compound of the general formula:

$R^7$-O-$R^3$-$Y^1$ (IV-2)

or

X-$R^3$-$Y^1$ (IV-3)

(wherein X represents a halogen atom and the other symbols are as hereinbefore defined) in an organic solvent such as dimethylformamide or tetrahydrofuran in the presence of a hydride of alkali metal such as sodium hydride at a temperature from ambient to 80°C.

When B represents a carbonyloxy group, it may be carried out by esterizing a compound of the general formula (IV) with a compound of the general formula:

HOOC-$R^3$-$Y^1$ (IV-4)

(wherein all the symbols are as hereinbefore defind) as mentioned above.

Step (c) may be carried out by reacting a compound of the general formula (IV) wherein B represents an oxygen atom, with a compound of the general formula:

$R^7$O-$R^3$-OTHP (IV-5)

or

$Y^1$-$R^3$-OTHP (IV-6)

(wherein all the symbols are as hereinbefore defined) under the same condition as described in step (c).

When B represents a carbonyloxy group, it may be carried out by esterizing a compound of the general formula (IV) with a compound of the general formula:

$HOOC-R^3-OTHP$ (IV-7)

(wherein all the symbols are as hereinbefore defined) as mentioned above.

Step (e) may be carried out by reacting a compound of the general formula (VII) with an alkylsulfonyl chloride such as mesyl chloride or with an arylsulfonyl chloride such as tosyl chloride at a temperature from -30°C to 50°C (i) in the presence of a tertiary amine such as pyridine or triethylamine, in an inert organic solvent such as methylene chloride or (ii) in pyridine.

The compound of the general formula (Ie') may be prepared as follows:

wherein B represents an oxygen atom, i.e. compound of the general formula:

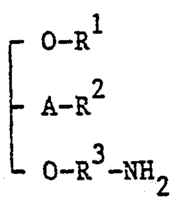

(Ie-1)

(wherein all the symbols are as hereinbefore defined) may be prepared by the reaction of the compound of the general formula:

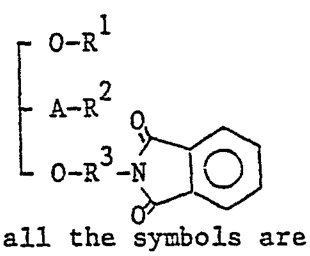

(Ie-2)

(wherein all the symbols are as hereinbefore defined) with hydrazine in a lower alkanol such as ethanol, and the compound of the general formula (Ie-2) may be prepared by the reaction of the compound of the general formula (IIa) or (IIIa) described above with phthalimide in an inert organic solvent such as dimethylformamide at a temperature from 50°C to a reflux temperature of the solvent.

wherein B represents a carbonyloxy group, i.e. compound of the general formula:

$$\left[\begin{array}{l} O-R^1 \\ A-R^2 \\ O-CO-R^3-NH_2 \end{array}\right. \qquad (Ie-3)$$

(wherein all the symbols are as hereinbefore defined) may be prepared by elimination of boc-group of the compound of the general formula:

$$\left[\begin{array}{l} O-R^1 \\ A-R^2 \\ O-CO-R^3-NH-boc \end{array}\right. \qquad (Ie-4)$$

(wherein boc represents tert-butoxycarbonyl group and the other symbols are as hereinbefore defined), and the compound of the general formula (Ie-4) may be prepared by esterification of the compound of the general formula:

$$HOOC-R^3-NH-boc \qquad (IV-8)$$

(wherein all the symbols are as hereinbefore defined and which is easily synthesized) with the compound of the general formula (IV) described above.

The reaction for eliminating boc group is well known, for example, it may be carried out by using an aqueous solution of hydrochloride in an inert organic solvent such as ethyl acetate, methylene chloride, chloroform, carbon tetrachloride or a lower alkanol at a temperature from ambient to 50°C, or by using trifluoroacetic acid at a temperature below ambient.

A compound of the general formula (V) wherein A represents a single bond, i.e. compound of the general formula:

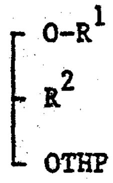

(Va)

(wherein all the symbols are as hereinbefore defined), may be prepared from malonic acid diethyl ester which is an already-known compound by the series of reactions depicted schematically below in Scheme II. In Scheme II, Et represents an ethyl group and the other symbols are as hereinbefore defined.

Scheme II

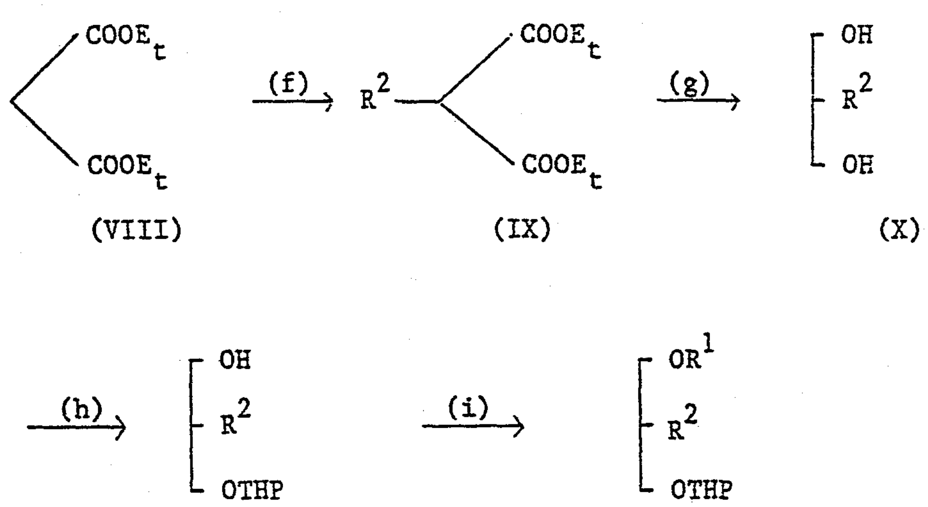


(VIII) (IX) (X)

(XI) (Va)

In Scheme II, each step can be effected by using methods known *per se*. For example, step (f) may be carried out by reacting a compound of the general formula (VIII) with a compound of the general formula:

$R^2$-$Y^1$ (VIII-1)

(wherein all the symbols are as hereinbefore defined) in the presence of a metal alkoxide prepared by using an alkali metal such as sodium in a low alkanol such as ethanol, at a temperature from ambient to a reflux temperature of the solvent.

Step (g) may be prepared by reacting a compound of the general formula (IX) with a useful metal hydride for reducing agent such as lithium aluminum hydride in an inert orgnic solvent such as ether or tetrahydrofuran at a temperature from ambient to a reflux temperature of the solvent.

Step (h) is a reaction for protecting by THP group, and may be carried out by using the same equivalent of 2.3-dihydropyran in methylene chloride in the presence of a small quantity of p-toluenesulfonic acid at ambient.

Step (i) may be carried out by reacting a compound of the general formula (XI) with a compound of the general formula:

$R^1$-X (XI-1)

(wherein all the symbols are as hereinbefore defined) under the same condition as described in Step (b) the method which a compound of the general formula (III) wherein B represents an oxygen atom may be prepared by

And a compound of the general formula (IV) wherein A represents a single bond in Scheme I, i.e. compound of the general formula:

O-$R^1$
$R^2$ (IVa)
OH

(wherein all the symbols are as hereinbefore defined) may be prepared by reacting a compound of the general formula (X) with a compound of the general formula (XI-1) under the same condition as described in step (i).

A compound of the general formula (V) wherein A represents an oxygen atom and a compound of the general formula (VI) wherein A and B represent an oxygen atom, i.e. compounds of the general formulae:

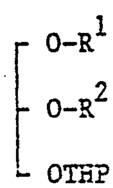

(Vb)

and

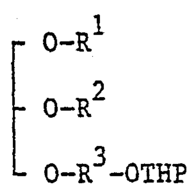

(VIb)

(wherein all the symbols are as hereinbefore defined) respectively, may be prepared by the series of reactions depicted schematically below in Scheme III. In Scheme III, all the symbols are as hereinbefore defined.

Scheme III

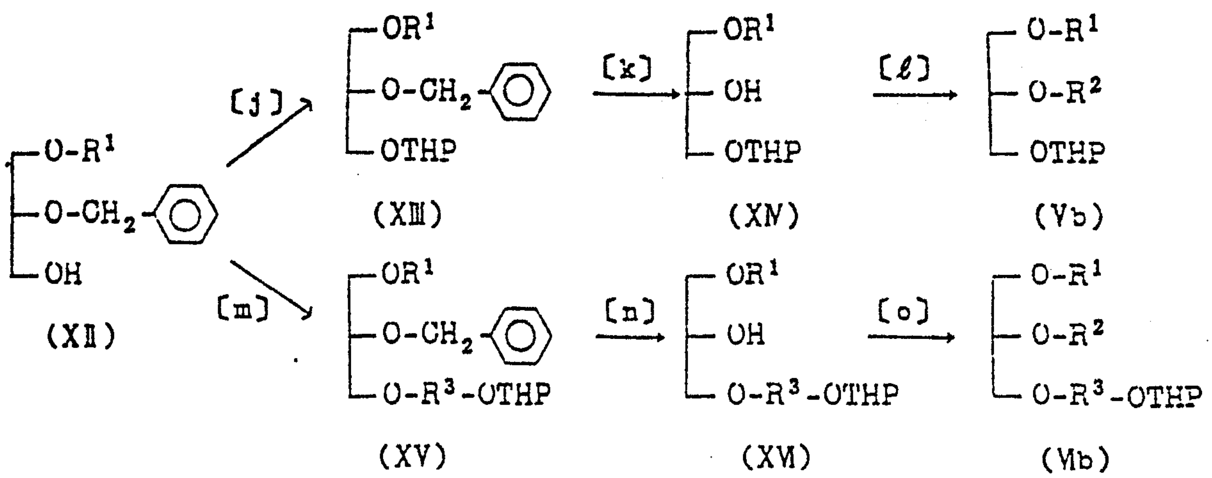


(XII) (XIII) (XIV) (Vb) (XV) (XVI) (VIb)

In Scheme III, each step can be effected by using methods known per se. For example, step (j) is a reaction for protecting by THP group, and may be carried out by the same condition as described in step (h).

Step (k) and (n) are a reaction for eliminating benzyl group, and such reaction may be carried out for example, in an inert organic solvent, e.g. a lower alkanol such as methanol or ethanol, ethyl acetate, acetic acid or the mixture of more than two of them in the presence of a catalyst of hydrogenation such as palladium on carbon, palladium on black or platinum two oxides under an atmosphere of hydrogen at a temperature from ambient to a reflux temperature of the solvent.

Step (l) or (o) may be carried out by reacting a compound of the general formula (XIV) or (XVI) with a compound of the general formula:

$R^7-O-R^2$ (XIV-1)

or

$X-R^2$ (XIV-2)

(wherein all the symbols are as hereinbefore defined) under the same condition as described in step (b).

Step (m) may be carried out by the same method as described in step (c).

A compound of the general formula (XII) may be prepared by the method described in the specification of our European Patent Publication No. 94586, but it may be also prepared by the series of reactions depicted schematically below in Scheme IV. In Scheme IV, all the symbols are as hereinbefore defined.

Scheme IV

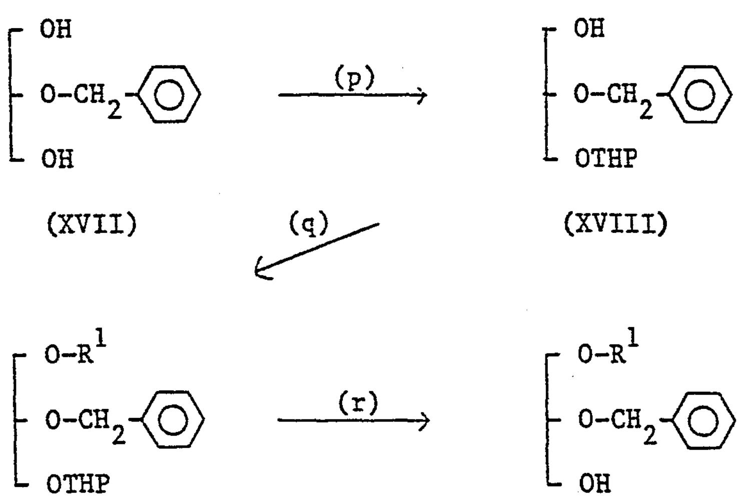


(XVII) (XVIII)

(XIX) (XII)

In Scheme IV, each step can be effected by using the same methods as mentioned above, i.e. step (p) may be carried out by the same method as described in step (h), step (q) may be (i) and step (r) may be (a).

According to a further feature of the present invention, a compound of the general formula (I) wherein $R^4$ represents a group of the formula $-\overset{\oplus}{N}(R^6)_3 \cdot \overset{\ominus}{Y}^1$ (in which all the symbols are hereinbefore defined), i.e. compound of the general formula:

$$\left[\begin{array}{l} O-R^1 \\ A-R^2 \\ B-R^3-\overset{\oplus}{N}(R^6)_3 \cdot \overset{\ominus}{Y}^1 \end{array}\right. \qquad \text{(If)}$$

(wherein all the symbols are as hereinbefore defined), may be also prepared by the reaction of a compound of the general formula:

$$\left[\begin{array}{l} O-R^1 \\ A-R^2 \\ B-R^3-N(R^6)_2 \end{array}\right. \qquad \text{(Ig)}$$

(wherein all the symbols are as hereinbefore defined) with an alkyl halide such as methyl iodide in an lower alkanol such as methanol or ethanol at a temperature from ambient to a reflux temperature of the solvent.

Compounds of the general formula (I) of the present invention, wherein $R^4$ represents a group of the formula: $\overset{\oplus}{N}(R^6)_3 \cdot \overset{\ominus}{Y}^3$ (in which $\overset{\ominus}{Y}^3$ is an anion of an acid other than $\overset{\ominus}{Y}^2$ and $R^6$ is as hereinbefore defined) may be easily prepared from a salt of a glycerol derivative of the general formula (I), wherein $R^4$ represents a group of the formula: $\overset{\oplus}{N}(R^6)_3 \cdot \overset{\ominus}{Y}^2$ by salt-exchange, for example, by neutralizing of a compound of the general formula (I), wherein $R^4$ represents a group of the formula: $\overset{\oplus}{N}(R^6)_3 \cdot \overset{\ominus}{Y}^2$ with an aqueous solution of sodium hydroxide, and then reacting with a desirable inorganic or organic acid or using an ion exchange resin.

Glycerol derivatives of the general formula (I), wherein $R^4$ represents a group of the formula: $-N(R^6)_2$ (wherein $R^6$ is as hereinbefore defined) may be converted into acid addition salts thereof. Preferably, acid addition salts are non-toxic salts and are water-soluble. Suitable acid addition salts are, for example, inorganic acid salts such as hydrochloride, hydrobromide, hydroiodide, sulfate, phosphorate, nitrate, or organic acid salts such as acetate, lactate, tartrate, benzoate, citrate, methanesulphonate, ethanesulphonate, benzenesulphonate, toluenesulphonate or isethionate. Acid addition salts may be prepared by methods known per se, for example, reacting stoichiometric quantities of a compound of the general formula (I), wherein $R^4$ represents an a group of the formula: $-N(R^6)_2$ and a desirable acid in a suitable solvent.

Glycerol derivatives of the general formula (I) and acid addition salts thereof have antagonistic effect on PAF, hypotensive effect like PAF, inhibitory effect on phospholipase and further, inhibitory effect on growth of tumour cell, and are, therefore, useful as a platelet aggregation inhibitor, anti-asthmatic agent, anti-allergic agent, anti-inflammatory agent, hypotensive agent and anti-tumour agent. These effect were confirmed by standard laboratory test and, for example, antagonistic effect on PAF was confirmed by the following screening system.

(1) Inhibitory effect on PAF-induced platelet aggregation

Whole blood of male guinea-pig was collected with a 3.8% aqueous solution of trisodium citrate in a proportion of 9:1 (v/v) and the mixture was centrifuged for 10 minutes at room temperature (120 x g) to obtain platelet rich plasma. Using obtained plasma, PAF

(10nM)-induced aggregation was determined by the absorbancy method using an aggregometer of Born (cf. J. Physiol., 162, 67 (1962)) .

The evaluation is conducted using the concentration of compounds of the present invention required to produce a 50% inhibition of each effect, i.e. $IC_{50}$. The results are shown in the following table.

Table 1

| Example No. of tested compounds | Inhibitory effect on PAF-induced platelet aggregation ($IC_{50}$, M) |
|---|---|
| 2 | $5.3 \times 10^{-6}$ |
| 5 | $1.3 \times 10^{-6}$ |
| 5(b) | $1.8 \times 10^{-6}$ |
| 7 | $3.8 \times 10^{-6}$ |

On the other hand, it was confirmed that the acute toxicity of all extent of the compounds of the present invention was more than 50 mg/kg by intravenous administration. Therefore, glycerol derivatives of the present invention may be considered to be sufficiently safe and suitable for medical use. For example, the mortality (dead/used) in glycerol derivatives of the present invention, i.e. compounds prepared in Example 2(b), 5, 5(a) and 5(b) was 2/5, 0/5, 0/5 and 0/5 respectively, when each glycerol compound was intravenously administered to tail vein in

ten mice at the dose of 50 mg/kg.

For the purpose hereinbefore described, glycerol derivatives of the general formula (I), or non-toxic acid addition salts thereof may be normally administered systemically or partially, usually by oral or parenteral administration. The dose to be administered is determined depending upon age, body weight, symptom, the desired therapeutic effect, the route of administration, and the duration of the treatment etc. In the human adult, the doses per person are generally between 1 mg and 1 g, preferably between 20 mg and 200 mg by oral administration up to several times per day, and between 100  g and 100 mg, preferably between 1 mg and 10 mg by parenteral administration up to several times per day.

As mentioned above, the doses to be used depend on various conditions. Therefore, there are cases in which doses lower than the ranges specified above and doses greater than the ranges specified above, may be used.

Solid compositions according to the present invention for oral administration include compressed tablets, dispersible powders and granules. In such solid compositions, one or more of the active compound(s) is, or are, admixed with at least one inert diluent such as lactose, mannitol, glucose, hydroxypropyl cellulose, microcrystalline cellulose, starch, polyvinylpyrrolidone or magnesium metasilicate aluminate. The compositions may also comprise, as is normal practice, additional substances other than inert diluents e.g. lubricating agents such as magnesium stearate, and, disintegrating agents such as

cellulose calcium gluconate. The tablets or pills may, if desired, be made into gastric film-coated or enteric film-coated tablets or pills, such as sugar-coated, gelatin-coated, hydroxy-propylcellulose-coated or hydroxypropylmethylcellulose phthalate-coated tablets or pills; two or more layers may be used. The compositions for oral administration also include capsules of absorbable material such as gelatin.

Liquid compositions for oral administration include pharmaceutically-acceptable emulsions, solutions, suspensions, syrups and elixirs containing inert diluents commonly used in the art such as distilled water or ethanol. Besides inert diluents such compositions may also comprise adjuvants such as wetting and suspending agents, and sweetening, flavouring, perfuming and preserving agents.

Other compositions for oral administration include spray compositions which may be prepared by known methods and which comprise one or more of the active compound(s).

Preparations for injection according to the present invention for parenteral administration include sterile aqueous or non-aqueous solutions, suspensions or emulsions. Examples of aqueous solvents or suspending media are distilled water for injection and physiological salt solution. Examples of non-aqueous solvents or suspending media are propylene glycol, polyethylene glycol, vegetable oils such as olive oil, alcohols such as ethanol, Polysorbate 80 (registered Trade Mark). These compositions may also include adjuvants such as preserving, wetting, emulsifying and dispersing agents. They may be

sterilized, for example, by filtration through a bacteria-retaining filter, by incorporation of sterilizing agents in the compositions or by irradiation. They may be also manufactured in the form of sterile solid compositions which can be dissolved in sterile water or some other sterile injectable medium immediately before use.

Other compositions for parenteral administration include liquids for external use, and endermic liniments such as ointments, suppositories for rectal administration and pessaries for vaginal administration which comprise one or more of the active compound(s) and may be prepared by known methods.

Preferred compounds of the general formula (I) of the present invention are, for example, as follows:

(2RS)-1-O-hexadecyl-2-dehydroxy-2-methyl-3-O-[7-(dimethylamino) heptyl] glycorol,

(2RS)-1-O-hexadecyl-2-dehydroxy-2-ethyl-3-O-[7-(dimethylamino) heptyl] glycorol,

(2RS)-1-O-hexadecyl-2-dehydroxy-2-propyl-3-O-[7-(dimethylamino) heptyl] glycorol,

(2RS)-1-O-hexadecyl-2-dehydroxy-2-butyl-3-O-[7-(dimethylamino) heptyl] glycorol,

(2RS)-1-O-hexadecyl-2-dehydroxy-2-pentyl-3-O-[7-(dimethylamino) heptyl] glycorol,

(2RS)-1-O-hexadecyl-2-dehydroxy-2-hexyl-3-O-[7-(dimethylamino) heptyl] glycorol,

(2RS)-1-O-hexadecyl-2-dehydroxy-2-allyl-3-O-[7-(dimethylamino) heptyl] glycorol,

(2RS)-1-O-hexadecyl-2-dehydroxy-2-methyl-3-O-[7-(trimethyl-ammonio)heptyl] glycerol·halide or its mesylate,

(2RS)-1-O-hexadecyl-2-dehydroxy-2-ethyl-3-O-[7-(trimethylammonio) heptyl] glycerol·halide or its mesylate,

(2RS)-1-O-hexadecyl-2-dehydroxy-2-propyl-3-O-[7-(trimethyl-ammonio)heptyl] glycerol·halide or its mesylate,

(2RS)-1-O-hexadecyl-2-dehydroxy-2-butyl-3-O-[7-(trimethylammonio) heptyl] glycerol·halide or its mesylate,

(2RS)-1-O-hexadecyl-2-dehydroxy-2-pentyl-3-O-[7-(trimethyl-ammonio)heptyl] glycerol·halide or its mesylate,

(2RS)-1-O-hexadecyl-2-dehydroxy-2-hexyl-3-O-[7-(trimethylammonio) heptyl] glycerol·halide or its mesylate,

(2RS)-1-O-hexadecyl-2-dehydroxy-2-allyl-3-O-[7-(trimethylammonio) heptyl] glycerol·halide or its mesylate,

(2RS)-1-O-hexadecyl-2-O-methyl-3-O-[7-(dimethylamino)heptyl] glycerol,

(2RS)-1-O-hexadecyl-2-O-ethyl-3-O-[7-(dimethylamino)heptyl] glycerol,

(2RS)-1-O-hexadecyl-2-O-propyl-3-O-[7-(dimethylamino)heptyl] glycerol,

(2RS)-1-O-hexadecyl-2-O-butyl-3-O-[7-(dimethylamino)heptyl] glycerol,

(2RS)-1-O-hexadecyl-2-O-pentyl-3-O-[7-(dimethylamino)heptyl] glycerol,

(2RS)-1-O-hexadecyl-2-O-hexyl-3-O-[7-(dimethylamino)heptyl] glycerol,

(2RS)-1-O-hexadecyl-2-O-allyl-3-O-[7-(dimethylamino)heptyl]
glycerol,

(2RS)-1-O-hexadecyl-2-O-methyl-3-O-[7-(trimethylammonio)heptyl]
glycerol•halide or its mesylate,

(2RS)-1-O-hexadecyl-2-O-ethyl-3-O-[7-(trimethylammonio)heptyl]
glycerol•halide or its mesylate,

(2RS)-1-O-hexadecyl-2-O-propyl-3-O-[7-(trimethylammonio)heptyl]
glycerol•halide or its mesylate,

(2RS)-1-O-hexadecyl-2-O-butyl-3-O-[7-(trimethylammonio)heptyl]
glycerol•halide or its mesylate,

(2RS)-1-O-hexadecyl-2-O-pentyl-3-O-[7-(trimethylammonio)heptyl]
glycerol•halide or its mesylate,

(2RS)-1-O-hexadecyl-2-O-hexyl-3-O-[7-(trimethylammonio)heptyl]
glycerol•halide or its mesylate,

(2RS)-1-O-hexadecyl-2-O-allyl-3-O-[7-(trimethylammonio)heptyl]
glycerol•halide or its mesylate,

and the compounds thereof wherein $R^3$ represents a trimethylen, tetramethylen, pentamethylen, hexamethylene or octamethylen group and the compounds thereof wherein B represents a carbonyloxy group.

The following Reference Examples and Examples illustrates, but not limit the preparation of compounds of the present invention. In the Reference Examples and Examples, 'TLC', 'NMR', 'IR' and 'Mass' represent 'Thin layer chromatography', 'Nuclear magnetic resonance', 'Infrared absorption spectrum' and 'Mass spectrum', respectively. The solvents specified in parentheses in chromatographic separations show the developing

solvent used. Except when specified otherwise, 'IR' is recorded by the liquid film method and 'NMR' is recorded in deuterochloroform ($CDCl_3$) solution.

Reference Example 1

n-propylmalonic acid diethyl ester

Under an atmosphere of argon, 3.5g of sodium metal and a solution of 5g of malonic acid diethyl ester on the market in 20ml of ethanol were added to 80ml of ethanol, and 4g of n-propyl bromide was added thereto and then the mixture was refluxed overnight. To the reaction mixture were added a dilute aqueous solution of hydrochloride and ice, and the mixture was extracted with ether. The extract was washed with a saturated aqueous solution of sodium bicarbonate and water, successively, dried and concentrated under reduced pressure. The residue was purified by column chromatography on silica gel (eluent; n-hexane : ethyl acetate = 8 : 1) to give 1.3g of the title compound having the following physical characteristics:

TLC(n-hexane : ethyl acetate = 5 : 1) : Rf = 0.54 ;

Mass : m/z = 203 ($M^+$ + 1), 160, 157.

By the same procedure as described in Reference Example 1, the following compounds were obtained by using (a) iso-propyl bromide, (b) n-pentyl bromide, (c) iso-pentyl bromide and (d) n-decyl bromide instead of n-propyl bromide used in Reference Example 1.

(a) iso-propylmalonic acid diethyl ester

TLC(n-hexane : ethyl acetate = 5 : 1) : Rf = 0.55;

Mass : m/z = 160, 157.

(b) n-pentylmalonic acid diethyl ester

TLC(n-hexane : ethyl acetate = 5 : 1) : Rf = 0.58;

Mass : m/z = 230 ($M^+$), 185, 173.

(c) iso-pentylmalonic acid diethyl ester

TLC(n-hexane : ethyl acetate = 5 : 1) : Rf = 0.61;

Mass : m/z = 231, 230 ($M^+$), 185, 173.

(d) n-decylmalonic acid diethyl ester

TLC(n-hexane : ethyl acetate = 5 : 1) : Rf = 0.61;

Mass : m/z = 300 ($M^+$), 255.

Reference Example 2

2-dehydroxy-2-n-propylglycerol

To 20ml of dry ether, 1.3g of n-propylmalonic acid diethyl ester (prepared in Reference Example 1) and 400mg of lithium aluminum hydride were added and the mixture was refluxed overnight. The reaction mixture was acidified by adding a dilute aqueous solution of hydrochloride and the mixture was filtered through the layer of celite-magnesium sulfate. The filtrate was concentrated under reduced pressure to give 820mg of the title compound having the following physical characteristic:

NMR : $\delta$ = 5.1 - 3.0 (6H, m), 2.2 - 0.5 (8H, m).

Reference Example 3

(2RS)-2-dehydroxy-2-n-propyl-3-O-(tetrahydropyran-2-yl)glycerol

To 10ml of methylene chloride, 390mg of 2-dehydroxy-2-n-propylglycerol (prepared in Reference Example 2), 252mg of 2,3-dihydropyran and 3mg of p-toluenesulfonic acid were added and the mixture was stirred for an hour at ambient. The

reaction mixture was neutralized by adding a saturated aqueous solution of sodium bicarbonate and the mixture was extracted with ethyl acetate. The extract was washed with water and a saturated aqueous solution of sodium chloride successively, dried and concentrated under reduced pressure. The residue was purified by column chromatography on silica gel (eluent ; n-hexane : ethyl acetate = 2 : 1) to give 199mg of the title compound having the following physical characteristics:

TLC(chloroform : acetone = 10 : 1) : Rf = 0.40 ;

NMR : $\delta$ = 4.5 (1H, m), 4.2 - 3.1 (6H, m), 2.8 (1H, m), 2.2 - 0.7 (14H, m).

Reference Example 4

(2RS)-1-O-hexadecyl-2-dehydroxy-2-n-propyl-3-O-(tetrahydropyran-2-yl) glycerol

Under an atmosphere of argon, to 10ml of N,N-dimethylformamide, 73mg of sodium hydride and 199mg of 2-n-propyl-3-O-(tetrahydropyran-2-yl)glycerol compound (prepared in Reference Example 3) were added. After the mixture was stirred for an hour at 60°C, cooled by ice water and 600mg of hexadecyl bromide was added thereto and the mixture was stirred for 10 minutes. After the mixture was warmed up to 70°C and stirred for 4 hours, 0.5ml of water was added thereto and the mixture was concentrated under reduced pressure. The residue was purified by column chromatography on silica gel (eluent ; n-hexane : ethyl acetate = 10 : 1) to give 510mg (impurities were included) of the title compound having the following physical characteristic:

TLC(n-hexane : ethyl acetate = 5 : 1) : Rf = 0.71.

By the same procedure as described in Reference Example 4, the following compound was obtained by using 2-n-propyl-glycerol compound instead of 2-n-propyl-3-O-(tetrahydropyran-2-yl)glycerol compound used in Reference Example 4.

(a) (2RS)-1-O-hexadecyl-2-dehydroxy-2-n-propylglycerol

Starting material : 2-dehydroxy-2-n-propylglycerol prepared in Reference Example 2 ;

TLC(n-hexane : ethyl acetate = 5 : 1) : Rf = 0.35 ;

NMR : δ = 3.7 - 3.1 (6H, m), 2.8 (1H, bs), 2.0 - 0.7 (39H, m) ;

IR : ν = 3400, 2930, 2860, 1460, 1110 $cm^{-1}$ ;

Mass : m/z = 342 ($M^+$), 324, 281.

Reference Example 5

(2RS)-1-O-hexadecyl-2-dehydroxy-2-n-propylglycerol

To 5ml of methanol, 2ml of methylene chloride, 5mg of p-toluenesulfonic acid and 510mg of 2-n-propyl-3-O-(tetrahydropyran-2-yl)glycerol compound (prepared in Reference Example 4) were added and the mixture was stirred at ambient for 2 hours. The reaction mixture was neutralized by adding a saturated aqueous solution of sodium bicarbonate and the mixture was concentrated under reduced pressure. The residue was purified by column chromatography on silica gel (eluent ; n-hexane : ethyl acetate = 8 : 1) to give 201mg of the title compound having the same physical characteristics as described in Reference Example 4 (a).

Reference Example 6

(2RS)-1-O-hexadecyl-2-dehydroxy-2-n-propyl-3-O-(4-chlorobutyl) glycerol

By the same procedure as described in Reference Example 4, 160mg of the title compound having the following physical characteristics was obtained by using 4-(p-toluenesulfonyloxy)butyl chloride instead of hexadecyl bromide used in Reference Example 4.

Starting material: (2RS)-1-O-hexadecyl-2-dehydroxy-2-n-propyl-glycerol prepared in Reference Example 5 ;

TLC(n-hexane : ethyl acetate = 5 : 1) : Rf = 0.74 ;

Mass : m/z = 432 ($M^+$), 324.

By the same procedure as described in Reference Example 6, the following compounds were obtained by using the various glycerol compounds prepared by the same procedure as described in Reference Example 2 to 5 and prepared from the compounds in Reference Example 1(a) to (d) as starting materials, instead of 2-n-propylglycerol compound used in Reference Example 6.

(a) (2RS)-1-O-hexadecyl-2-dehydroxy-2-iso-propyl-3-O-(4-chlorobutyl)glycerol

Starting material : (2RS)-1-O-hexadecyl-2-dehydroxy-2-iso-propylglycerol;

TLC(n-hexane : ethyl acetate = 5 : 1) : Rf = 0.75.

(b) (2RS)-1-O-hexadecyl-2-dehydroxy-2-n-pentyl-3-O-(4-chlorobutyl)glycerol

Starting material : (2RS)-1-O-hexadecyl-2-dehydroxy-2-n-pentylglycerol;

TLC(n-hexane : ethyl acetate = 5 : 1) : Rf = 0.75.

(c) (2RS)-1-O-hexadecryl-2-dehydroxy-2-iso-pentyl-3-O-(4-chlorobutyl)glycerol

Starting material : (2RS)-1-O-hexadecyl-2-dehydroxy-2-iso-pentylglycerol ;

TLC(n-hexane : ethyl acetate = 5 : 1) : Rf = 0.75.

(d) (2RS)-1-O-hexadecyl-2-dehydroxy-2-n-decyl-3-O-(4-chlorobutyl) glycerol

Starting material : (2RS)-1-O-hexadacyl-2-dehydroxy-2-n-decylglycerol;

TLC(n-hexane : ethyl acetate = 5 : 1) : Rf = 0.75.

Example 1

(2RS)-1-O-hexadecyl-2-dehydroxy-2-n-propyl-3-O-[4-(dimethylamino) butyl]glycerol

To 6ml of N, N-dimethylformamide, 160mg of 2-n-propyl-3-O-(4-chlorobutyl)glycerol compound (prepared in Reference Example 6) and 1.3ml of 40% aqueous solution of dimethylamine were added and the mixture was stirred at 65°C overnight. After the reaction mixture was concentrated under reduced pressure, the residue was purified by column chromatography on silica gel (eluent ; chloroform : methanol : triethylamine = 30 : 1 : 0.05) to give 64mg of the title compound having the following physical characteristics:

TLC(ethyl acetate : acetic acid : water = 3 : 1 : 1) : Rf = 0.54;

NMR : $\delta$ = 3.5 - 3.0 (8H, m), 2.2 (6H, s), 2.4 - 2.0 (2H, m), 1.9 - 0.6 (43H, m);

IR : ν = 2930, 2860, 1460, 1110 $cm^{-1}$;

Mass : 441 ($M^+$), 216.

By the same procedure as described in Example 1, the following compounds were obtained by using the various glycerol compounds prepared in Reference Example 6 (a) to (d), instead of 2-n-propyl-3-O-(4-chlorobutyl)glycerol compound.

(a) (2RS)-1-O-hexadecyl-2-dehydroxy-2-iso-propyl-3-O-[4-(dimethylamino)butyl]glycerol

Starting material : (2RS)-1-O-hexadecyl-2-dehydroxy-2-iso-propyl-3-O-(4-chlorobutyl)glycerol prepared in Reference Example 6 (a);

TLC(ethyl acetate : acetic acid : water = 3 : 1 : 1) : Rf = 0.57;

NMR : δ = 3.6 - 3.2 (8H, m), 2.26 (6H, s), 2.5 - 2.2 (2H, m), 2.0 - 0.7 (39H, m);

IR : ν = 2920, 2850, 1460, 1110 $cm^{-1}$;

Mass : m/z = 441 ($M^+$), 426, 398.

(b) (2RS)-1-O-hexadecyl-2-dehydroxy-2-n-pentyl-3-O-[4-(dimethylamino)butyl]glycerol

Starting material : (2RS)-1-O-hexadecyl-2-dehydroxy-2-n-pentyl-3-O-(4-chlorobutyl)glycerol prepared in Reference Example 6 (b);

TLC(ethyl acetate : acetic acid : water = 3 : 1 : 1) : Rf = 0.57;

NMR : δ = 3.6 - 3.1 (8H, m), 2.2 (6H, s), 2.5 - 2.0 (2H, m), 2.0 - 0.7 (43H, m);

IR : ν = 2920, 2840, 1460, 1110 $cm^{-1}$;

Mass : m/z = 469 ($M^+$), 244.

(c) (2RS)-1-O-hexadecyl-2-dehydroxy-2-iso-pentyl-3-O-[4-(dimethylamino)butyl]glycerol

Starting material : (2RS)-1-O-hexadecyl-2-dehydroxy-2-iso-pentyl-3-O-(4-chlorobutyl)glycerol prepared in Reference Example 6 (c) ;

TLC(ethyl acetate : acetic acid : water = 3 : 1 : 1) : Rf = 0.57 ;

NMR : δ = 3.6 - 3.1 (8H, m), 2.2 (6H, s), 2.5 - 2.0 (2H, m), 1.9 - 0.7 (43H, m) ;

IR : ν = 2930, 2850, 1460, 1110 $cm^{-1}$ ;

Mass : m/z = 469 ($M^+$), 454, 244.

(d) (2RS)-1-O-hexadecyl-2-dehydroxy-2-n-decyl-3-O-[4-(dimethylamino)butyl]glycerol

Starting material : (2RS)-1-O-hexadecyl-2-dehydroxy-2-n-decyl-3-O-(4-chlorobutyl)glycerol prepared in Reference Example 6 (d) ;

TLC(ethyl acetate : acetic acid : water = 3 : 1 : 1) : Rf = 0.60 ;

NMR : δ = 3.6 - 3.1 (8H, m), 2.26 (6H, s), 2.6 - 2.1 (2H, m), 2.0 - 0.7 (57H, m) ;

IR : ν = 2920, 2850, 1460, 1110 $cm^{-1}$ ;

Mass : m/z = 539 ($M^+$), 314.

Example 2

(2RS)-1-O-hexadecyl-2-dehydroxy-2-n-propyl-3-O-[4-(trimethylammonio)butyl]glycerol·iodide

To 3ml of methanol, 41mg of 2-n-propyl-3-O-[4-(dimethylamino)butyl]glycerol compound (prepared in Example 1), 0.5ml of methyl iodide and about 5mg of potassium bicarbonate were added and the mixture was stirred at ambient overnight. After the reaction mixture was concentrated under reduced pressure and dried, chloroform was added to the residue and the mixture was filtered. The filtrate was concentrated under reduced pressure to give 52mg of the title compound having the following physical characteristics:

Melting point : 127 - 133°C ;

TLC(ethyl acetate : acetic acid : water = 3 : 1 : 1) :

Rf = 0.41 ;

NMR : δ = 3.72 - 3.58 (2H, m), 3.44 (9H, s),
3.54 - 3.26 (8H, m), 2.00 - 1.45 (7H, m),
1.26 (30H, m), 1.00 - 0.80 (6H, m) ;

IR (KBr method) : ν = 3450, 2930, 2850, 1470, 1120 $cm^{-1}$ ;

Mass : m/z = 441, 324, 216, 183.

By the same procedure as described in Example 2, the following compound was obtained by using ethyl iodide instead of methyl iodide used in Example 2.

(a) (2RS)-1-O-hexadecyl-2-dehydroxy-2-n-propyl-3-O-[4-(N,N-dimethyl-N-ethylammonio)butyl]glycerol iodide.

Starting material : 2-n-propyl-3-O- 4-(dimethylamino)butyl glycerol compound prepared in Example 1 ;

Melting point : 49 - 55°C ;

TLC(ethyl acetate : acetic acid : water = 3 : 1 : 1) :

Rf = 0.41 ;

NMR : δ = 3.80 - 3.28 (9H, m), 3.35 (9H, s),
1.96 - 1.50 (7H, m), 1.50 - 1.20 (33H, m),
0.98 - 0.82 (6H, m) ;

IR(KBr method) : ν = 3450, 2930, 2850, 1470, 1120 $cm^{-1}$ ;

Mass : m/z = 455, 440, 324, 230.

By the same procedure as described in Example 2, the following compounds were obtained by using the various glycerol compounds prepared in Example 1 (a) to (d) instead of 2-n-propyl-3-O-[4-(dimethylamino)butyl]glycerol compound used in Example 2.

(b) (2RS)-1-O-hexadecyl-2-dehydroxy-2-iso-propyl-3-O-[4-(trimethylammonio)butyl]glycerol•iodide

Starting material : (2RS)-1-O-hexadecyl-2-dehydroxy-2-iso-propyl-3-O-[4-(dimethylamino)butyl]glycerol prepared in Example 1 (a) ;

Melting point : 140 - 143°C

TLC(ethyl acetate : acetic acid : water = 3 : 1 : 1) : Rf = 0.55 ;

NMR : δ = 3.72 - 3.57 (2H, m), 3.44 (9H, s),
3.54 - 2.28 (8H, m), 2.00 - 1.46 (8H, m),
1.24 (26H, m), 0.90 (6H, d), 0.87 (3H, m) ;

IR (KBr method) : ν = 3450, 2920, 2850, 1470, 1120 $cm^{-1}$ ;

Mass : m/z = 441, 398, 216.

(c) (2RS)-1-O-hexadecyl-2-dehydroxy-2-n-pentyl-3-O-[4-(trimethylammonio)butyl]glycerol•iodide

Starting material : (2RS)-1-O-hexadecyl-2-dehydroxy-2-n-

pentyl-3-O-[4-(dimethylamino)butyl]glycerol prepared in Example 1 (b) ;

Melting point : 58 - 60°C ;

TLC(ethyl acetate : acetic acid : water = 3 : 1 : 1) : Rf = 0.57 ;

NMR : δ = 3.70 - 3.57 (2H, m), 3.44 (9H, s), 3.60 - 3.27 (8H, m), 2.00 - 1.45 (7H, m), 1.25 (34H, m), 0.87 (6H, m) ;

IR : ν = 3400, 2930, 2850, 1460, 1110 $cm^{-1}$ ;

Mass : m/z = 469, 244.

(d) (2RS)-1-O-hexadecyl-2-dehydroxy-2-iso-pentyl-3-O-[4-(trimethylammonio)butyl]glycerol•iodide

Starting material : (2RS)-1-O-hexadecyl-2-dehydroxy-2-iso-pentyl-3-O-[4-(dimethylamino)butyl]glycerol prepared in Example 1 (c) ;

Melting point : 70 - 78°C;

TLC(ethyl acetate : acetic acid : water = 3 : 1 : 1) : Rf = 0.57 ;

NMR : δ = 3.72 - 3.57 (2H, m), 3.45 (9H, s), 3.54 - 3.28 (8H, m), 2.00 - 1.42 (8H, m), 1.26 (30H, m), 0.89 (6H, d), 0.86 (3H, m) ;

IR(KBr method) : ν = 3450, 2920, 2850, 1460, 1120 $cm^{-1}$ ;

Mass : m/z = 469, 454, 426, 244.

(e) (2RS)-1-O-hexadecyl-2-dehydroxy-2-n-decyl-3-O-[4-(trimethylammonio)butyl]glycerol•iodide

Starting material : (2RS)-1-O-hexadecyl-2-dehydroxy-2-n-decyl-3-O-[4-(dimethylamino)butyl]glycerol prepared

in Example 1 (d) ;

TLC(ethyl acetate : acetic acid : water = 3 : 1 : 1) :

Rf = 0.58 ;

NMR : δ = 3.70 - 3.56 (2H, m), 3.43 (9H, s),

3.60 - 3.25 (8H, m), 1.98 - 1.24 (7H, m),

1.25 (44H, m), 0.87 (6H, m) ;

IR : ν = 2920, 2850, 1460, 1110 $cm^{-1}$ ;

Mass : m/z = 539, 314.

Reference Example 7

(2RS)-1-O-hexadecyl-2-O-benzyl-3-O-(tetrahydropyran-2-yl)glycerol

By the same procedure as described in Reference Example 3, the title compound having th following physical characteristic was obtained by using (2RS)-1-O-hexadecyl-2-O-benzylglycerol instead of 2-n-propylglycerol compound used in Reference Example 3.

Starting material : (2RS)-1-O-hexadecyl-2-O-benzylglycerol ;

TLC(n-hexane : ethyl acetate = 5 : 1) : Rf = 0.47.

Reference Example 8

(2RS)-1-O-hexadecyl-3-O-(tetrahydropyran-2-yl)glycerol

To 10ml of ethanol, 356mg of benzylglycerol compound (prepared in Reference Example 7) and 300mg of 10% palladium on carbon were added and the mixture was stirred with browing hydrogen gas for 2 hours at ambient. After the reaction was over, the catalyst was filtered and the filtrate was concentrated under reduced pressure to give 262mg of the title compound having the following physical characteristic :

TLC(n-hexane : ethyl acetate = 5 : 1) : Rf = 0.30.

Reference Example 9

(2RS)-1-O-hexadecyl-2-O-ethyl-3-O-(tetrahydropyran-2-yl)glycerol

By the same procedure as described in Reference Example 4, the title compound having the following physical characteristics was obtained by using ethyl iodide instead of hexadecyl bromide used in Reference Example 4.

Starting material : (2RS)-1-O-hexadecyl-3-O-(tetrahydropyran-2-yl)glycerol prepared in Reference Example 8 ;

TLC(n-hexane : ethyl acetate = 5 : 1) : Rf = 0.48 ;

NMR : $\delta$ = 4.7 - 4.5 (1H, m), 4.0 - 3.2 (11H, m), 2.0 - 0.6 (40H, m).

Reference Example 10

(2RS)-1-O-hexadecyl-2-O-ethylglycerol

By the same procedure as described in Reference Example 5, the title compound having the following physical characteristics was obtained by using (2RS)-1-O-hexadecyl-2-O-ethyl-3-O-(tetrahydropyran-2-yl)glycerol (prepared in Reference Example 9) instead of 2-n-propyl-3-O-(tetrahydropyran-2-yl)glycerol compound used in Reference Example 5.

Starting material : (2RS)-1-O-hexadecyl-2-O-ethyl-3-O-(tetrahydropyran-2-yl)glycerol prepared in Reference Example 9 ;

TLC(n-hexane : ethyl acetate = 5 : 1) : Rf = 0.18 ;

NMR : $\delta$ = 3.9 - 3.1 (9H, m), 2.3 (1H, bs),
1.7 - 0.6 (34H, m) ;

IR : $\nu$ = 3450, 2920, 2850, 1460, 1110 $cm^{-1}$.

Reference Example 11

(2RS)-1-O-hexadecyl-2-O-ethyl-3-O-(4-chlorobutyl)glycerol

By the same procedure as described in Reference Example 4, the title compound having the following pysical characteristics was obtained by using 4-(p-toluenesulfonyloxy)butyl chloride instead of hexadecyl bromide used in Reference Example 4.

Starting material : (2RS)-1-O-hexadecyl-2-O-ethylglycerol prepared in Reference Example 10 ;

TLC(n-hexane : ethyl acetate = 5 : 1) : Rf = 0.55 ;

NMR : $\delta$ = 3.9 - 3.1 (13H, m), 2.0 - 0.6 (38H, m) ;

Mass : m/z = 434 ($M^+$), 399, 388.

Example 3

(2RS)-1-O-hexadecyl-2-O-ethyl-3-O-[4-(dimethylamino)butyl]glycerol

By the same procedure as described in Example 1, the title compound having the following physical characteristics was obtained by using 2-O-ethyl-3-O-(4-chlorobutyl)glycerol compound (prepared in Reference Example 11) instead of 2-n-propyl-3-O-(4-chlorobutyl)glycerol compound used in Example 1.

Starting material : (2RS)-1-O-hexadecyl-2-O-ethyl-3-O-(4-chlorobutyl)glycerol prepared in Reference Example 11 ;

TLC(ethyl acetate : acetic acid : water = 3 : 1 : 1) :

Rf = 0.58 ;

NMR : δ = 3.58 (2H, q), 3.60 - 3.10 (9H, m),

2.40 - 2.10 (2H, m), 2.16 (6H, s),

1.80 - 0.60 (38H, m) ;

Mass : m/z = 443 ($M^+$), 428, 413, 398.

Example 4

(2RS)-1-O-hexadecyl-2-O-ethyl-3-O-[4-(trimethylammonio)butyl] glycerol iodide

By the same procedure as described in Example 2, the title compound having the following physical characteristics was obtained by using 2-O-ethyl-3-O-[4-(dimethylamino)butyl]glycerol compound (prepared in Example 3) instead of 2-n-propyl-3-O-4-(dimethylamino)butyl glycerol compound used in Example 2.

Starting material : (2RS)-1-O-hexadecyl-2-O-ethyl-3-O-[4-(dimethylamino)butyl]glycerol prepared in Example 3 ;

Melting point : 48 - 52°C ;

TLC(ethyl acetate : acetic acid : water = 3 : 1 : 1) :

Rf = 0.52 ;

NMR : δ = 3.72 - 3.36 (13H, m), 3.43 (9H, s),

2.00 - 1.80 (2H, m), 1.80 - 1.45 (4H, m),

1.25 (26H, m), 1.18 (3H, t), 0.88 (3H, m) ;

IR : ν = 3540, 2980, 2850, 1465, 1110 $cm^{-1}$ ;

Mass : m/z = 443, 428, 414, 398.

Reference Example 12

(2RS)-1-O-hexadecyl-2-O-benzyl-3-O-[7-(tetrahydropyran-2-yloxy)heptyl]glycerol

By the same procedure as described in Reference Example 4, the title compound having the following physical characteristics was obtained by using 2-[7-(p-toluenesulfonyloxy)heptyloxy]tetrahydropyran instead of hexadecyl bromide used in Reference Example 4.

Starting material : (2RS)-1-O-hexadecyl-2-O-benzylglycerol ;

TLC(n-hexane : ethyl acetate = 5 : 1) : Rf = 0.55 ;

NMR : $\delta$ = 7.2 (5H, s), 4.6 (2H, s), 4.6 - 4.3 (1H, m),
4.0 - 3.0 (13H, m), 2.0 - 0.6 (47H, m) ;

Mass : m/z = 604 ($M^+$), 519.

By the same procedure as described in Reference Example 12, the following compound was obtained by using 2-[4-(p-toluenesulfonyloxy)butyloxy]tetrahydropyran instead of 2-[7-(p-toluenesulfonyloxy)heptyloxy]tetrahydropyran used in Reference Example 12.

(a) (2RS)-1-O-hexadecyl-2-O-benzyl-3-O-[4-(tetrahydropyran-2-yloxy)butyl]glycerol

Starting material : (2RS)-1-O-hexadecyl-2-O-benzylglycerol ;

TLC(n-hexane : ethyl acetate = 5 : 1) : Rf = 0.50 ;

NMR : $\delta$ = 7.2 (5H, s), 4.6 (2H, s), 4.6 - 4.4 (1H, m),
3.9 - 3.1 (13H, m), 2.0 - 0.7 (41H, m) ;

Mass : m/z = 562 ($M^+$), 477, 471.

Reference Example 13

(2RS)-1-O-hexadecyl-3-O-[7-(tetrahydropyran-2-yloxy)heptyl]glycerol

By the same procedure described in Reference Example 8, the title compound having the following physycal characteristics was obtained by using 2-O-benzyl-3-O-[7-(tetrahydropyran-2-yloxy)heptyl]glycerol compound prepared in Reference Example 12 instead of 2-O-benzyl-3-O-(tetrahydropyran-2-yl)glycerol compound used in Reference Example 8.

Starting material : (2RS)-1-O-hexadecyl-2-O-benzyl-3-O-[7-(tetrahydropyran-2-yloxy)heptyl]glycerol prepared in Reference Example 12 ;

TLC(n-hexane : ethyl acetate = 5 : 1) : Rf = 0.27 ;

NMR : $\delta$ = 4.7 - 4.4 (1H, m), 4.1 - 3.1 (13H, m), 2.7 - 2.3 (1H, m), 2.0 - 0.7 (47H, m);

IR : $\nu$ = 3500, 2930, 2850, 1460, 1120 $cm^{-1}$ ;

Mass : m/z = 514 ($M^+$), 496, 431, 429, 411.

By the same procedure as described in Reference Example 13, the compound having the following physical characteristics was obtained by using 2-O-benzyl-3-O-butylglycerol compound instead of 2-O-benzyl-3-O-heptylglycerol compound used in Reference Example 13.

(a) (2RS)-1-O-hexadecyl-3-O-[4-(tetrahydropyran-2-yl)butyl]glycerol

Starting material : (2RS)-1-O-hexadecyl-2-O-benzyl-3-O-[4-(tetrahydropyran-2-yloxy)butyl]glycerol prepared in Reference Example 12 (a) ;

TLC(n-hexane : ethyl acetate = 5 : 1) : Rf = 0.25 ;

NMR : $\delta$ = 4.6 - 4.4 (1H, m), 3.9 - 3.1 (13H, m),
2.0 - 0.7 (42H, m) ;

IR : $\nu$ = 3450, 2930, 2850, 1470, 1120 $cm^{-1}$ ;

Mass : m/z = 472 ($M^+$), 387.

Reference Example 14

(2RS)-1-O-hexadecyl-2-O-methyl-3-O-[7-(tetrahydropyran-2-yloxy)heptyl]glycerol

By the same procedure as described in Reference Example 4, the title compound having the following physical characteristics was obtained by using methyl iodide instead of hexadecyl bromide used in Reference Example 4. (with the proviso that the obtained compound was not purified by column chromatography on silica gel.)

Starting material : (2RS)-1-O-hexadecyl-3-O-[7-(tetrahydropyran-2-yloxy)heptyl]glycerol prepared in Reference Example 13 ;

TLC(n-hexane : ethyl acetate = 5 : 1) : Rf = 0.46;

NMR : $\delta$ = 4.6 - 4.4 (1H, m), 4.0 - 3.0 (16H, m),
2.0 - 0.6 (47H, m);

Mass : m/z = 528 ($M^+$), 513, 496, 445, 443, 411.

By the same procedure as described in Reference Example 14, the following compounds were obtained by using (a) ethyl iodide or (b) propyl iodide instead of methyl iodide used in Reference Example 14.

(a) (2RS)-1-O-hexadecyl-2-O-ethyl-3-O-7-(tetrahydropyran-2-yloxy)

heptyl glycerol

TLC(n-hexane : ethyl acetate = 5 : 1) : Rf = 0.52;

NMR : δ = 4.6 - 4.4 (1H, m), 4.0 - 3.1 (15H, m),
2.0 - 0.6 (50H, m);

Mass : m/z = 542 ($M^+$), 513, 496, 459, 457, 411.

(b) (2RS)-1-O-hexadecyl-2-O-n-propyl-3-O-[7-(tetrahydropyran-2-yloxy)heptyl]glycerol

TLC(n-hexane : ethyl acetate = 5 : 1) : Rf = 0.55;

NMR : δ = 4.6 - 4.4 (1H, m), 3.9 - 3.1 (15H, m),
2.0 - 0.6 (52H, m);

Mass : m/z = 556 ($M^+$), 496, 473, 471, 411.

By the same procedure as described in Reference Example 14, the following compounds were obtained by using 3-O-butylglycerol compound prepared in Reference Example 13 (a) instead of 3-O-heptylglycerol compound and using (c) allyl iodide or (d) hexadecyl iodide instead of methyl iodide used in Reference Example 14.

(c) (2RS)-1-O-hexadecyl-2-O-allyl-3-O-[4-(tetrahydropyran-2-yloxy)butyl]glycerol

Starting material : (2RS)-1-O-hexadecyl-3-O-[4-(tetrahydropyran-2-yloxy)butyl]glycerol prepared in Reference Example 13 (a);

TLC(n-hexane : ethyl acetate = 5 : 1) : Rf = 0.49;

NMR : δ = 6.17 - 5.47 (1H, m), 5.47 - 4.93 (2H, m),
4.60 - 4.40 (1H, m), 4.28 - 4.00 (2H, m),
3.90 - 3.10 (13H, m), 2.00 - 0.70 (41H, m);

IR : $\nu$ = 2930, 2850, 1465, 1120 $cm^{-1}$ ;

Mass : m/z = 518 ($M^+$), 477.

(d) (2RS)-1-O-hexadecyl-2-O-hexadecyl-3-O-[4-(tetrahydropyran-2-yloxy)butyl]glycerol

Starting material : (2RS)-1-O-hexadecyl-3-O-[4-(tetrahydropyran-2-yloxy)butyl]glycerol prepared in Reference Example 13 (a);

TLC(n-hexane : ethyl acetate = 5 : 1) : Rf = 0.65;

NMR : $\delta$ = 4.6 - 4.4 (1H, m), 4.0 - 3.0 (15H, m), 2.0 - 0.7 (72H, m);

Mass : m/z = 696 ($M^{\dot{+}}$), 611.

Reference Example 15

(2RS)-1-O-hexadecyl-2-O-methyl-3-O-(7-hydroxyheptyl)glycerol

By the same procedure as described in Reference Example 5, the title compound having the following physical characteristic was obtained by using 2-O-methylglycerol compound (prepared in Reference Example 14) instead of 2-n-propylglycerol compound used in Reference Example 5. (with the proviso that the obtained compound was not purified by column chromatography on silica gel)

Starting material : (2RS)-1-O-hexadecyl-2-O-methyl-3-O-[7-(tetrahydropyran-2-yloxy)heptyl]glycerol prepared in reference Example 14;

TLC(n-hexane : ethyl acetate = 5 : 1) : Rf = 0.10.

By the same procedure as described in Reference Example 15, the following compounds were obtained by using the various

glycerol compounds prepared in Reference Example 14 (a) to (d) instead of 2-O-methylglycerol compound used in Reference Example 15.

(a) (2RS)-1-O-hexadecyl-2-O-ethyl-3-O-(7-hydroxyheptyl)glycerol

Starting material : (2RS)-1-O-hexadecyl-2-O-ethyl-3-O-[7-(tetrahydropyran-2-yloxy)heptyl]glycerol prepared in Reference Example 14 (a);

TLC(n-hexane : ethyl acetate = 5 : 1) : Rf = 0.16.

(b) (2RS)-1-O-hexadecyl-2-O-n-propyl-3-O-(7-hydroxyheptyl) glycerol

Starting Material : (2RS)-1-O-hexadecyl-2-O-n-propyl-3-O-[7-(tetrahydropyran-2-yloxy)heptyl]glycerol prepared in Reference Example 14 (b);

TLC(n-hexane : ethyl acetate = 5 : 1) : Rf = 0.19.

(c) (2RS)-1-O-hexadecyl-2-O-allyl-3-O-(4-hydroxybutyl)glycerol

Starting material : (2RS)-1-O-hexadecyl-2-O-allyl-3-O-[4-(tetrahydropyran-2-yloxy)butyl]glycerol prepared in Reference Example 14 (c);

TLC(n-hexane : ethyl acetate = 5 : 1) : Rf = 0.19;

NMR : $\delta$ = 6.2 - 5.5 (1H, m), 5.5 - 4.9 (2H, m), 4.3 - 4.0 (2H, m), 3.9 x 3.0 (11H, m), 2.0 - 0.7 (36H, m);

IR : $\nu$ = 3450, 2930, 2850, 1470, 1120 $cm^{-1}$.

(d) (2RS)-1-O-hexadecyl-2-O-hexadecyl-3-O-(4-hydroxybutyl) glycerol

Starting material : (2RS)-1-O-hexadecyl-2-O-hexadecyl-3-O-

[4-(tetrahydropyran-2-yloxy)butyl]glycerol prepared in Reference Example 14 (d);

TLC(n-hexane : ethyl acetate = 5 : 1) : Rf = 0.23;

NMR : $\delta$ = 4.0 - 3.0 (13H, m), 2.0 - 0.7 (67H, m);

IR : $\nu$ = 3450, 2920, 2850, 1470, 1120 $cm^{-1}$.

Reference Example 16

(2RS)-1-O-hexadecyl-2-O-methyl-3-O-[7-(methanesulfonyloxy)heptyl] glycerol

To 10ml of methylene chloride, 280mg of (2RS)-1-O-hexadecyl-2-O-methyl-3-O-(7-hydroxyheptyl)glycerol prepared in Reference Example 15, 0.5ml of an aqueous solution of triethylamine and 70ml of methanesulfonyl chloride were added, and the mixture was stirred for 30 minutes at 0°C. To the reaction mixture, water was added and extracted with ethyl acetate and the extract was washed with water and a saturated aqueous solution of sodium chloride successively, dried and concentrated under reduced pressure. The residue was purified by column chromatography on silica gel (eluent; n-hexane : ethyl acetate = 5 : 1) to give 270mg of the title compound having the following physical characteristics;

TLC(chloroform : acetone = 10 : 1) : Rf = 0.83;

NMR : $\delta$ = 4.16 (2H, t), 3.80 - 3.20 (12H, m),

2.96 (3H, s), 2.00 - 0.70 (41H, m);

Mass : m/z = 522 ($M^+$), 490.

By the same procedure as described in Reference Example 16, the following compounds were obtained by using the various

glycerol compounds prepared in Reference Example 15 instead of 2-O-methylglycerol compound used in Reference Example 16.

(a) (2RS)-1-O-hexadecyl-2-O-ethyl-3-O-[7-(methanesulfonyloxy) heptyl]glycerol

Starting material : (2RS)-1-O-hexadecyl-2-O-ethyl-3-O-(7-hydroxyheptyl)glycerol prepared in Reference Example 15 (a);

TLC(chloroform : acetone = 10 : 1) : Rf = 0.84;

NMR : δ = 4.16 (2H, t), 3.58 (2H, q),
3.60 - 3.20 (9H, m), 2.96 (3H, s),
2.00 - 0.70 (44H, m);

Mass : m/z = 537, 536 ($M^+$), 507, 490.

(b) (2RS)-1-O-hexadecyl-2-O-n-propyl-3-O-[7-(methanesulfonyloxy) heptyl]glycerol

Starting material : (2RS)-1-O-hexadecyl-2-O-n-propyl-3-O-(7-hydroxyheptyl)glycerol prepared in Reference Example 15 (b);

TLC(chloroform : acetone = 10 : 1) : Rf = 0.88;

NMR : δ = 4.16 (2H, t), 3.80 - 3.20 (11H, m),
2.96 (3H, s), 2.00 - 0.70 (46H, m);

Mass : m/z = 551, 550 ($M^+$), 507, 490.

(c) (2RS)-1-O-hexadecyl-2-O-allyl-3-O-[4-(methanesulfonyloxy) butyl]glycerol

Starting material : (2RS)-1-O-hexadecyl-2-O-allyl-3-O-(4-hydroxybutyl)glycerol prepared in Reference Example 15 (c);

TLC(chloroform : acetone = 10 : 1) : Rf = 0.81;

NMR : δ = 6.2 - 5.5. (1H, m), 5.5 - 4.9 (2H, m),
4.2 (2H, t), 4.3 - 4.0 (2H, m),
3.9 - 3.0 (9H, m), 2.9 (3H, s),
2.0 - 0.7 (35H, m).

(d) (2RS)-1-O-hexadecyl-2-O-hexadecyl-3-O-[4-(methanesulfonyloxy) butyl]glycerol

Starting material : (2RS)-1-O-hexadecyl-2-O-hexadecyl-3-O-(4-hydroxybutyl)glycerol prepared in Reference Example 15 (d);

TLC(chloroform : acetone = 10 : 1) : Rf = 0.86;

NMR : δ = 4.2 (2H, t), 4.0 - 3.1 (11H, m),
3.0 (3H, s), 2.0 - 0.7 (66H, m).

Example 5

(2RS)-1-O-hexadecyl-2-O-methyl-3-O-[7-(trimethylammonio)heptyl] glycerol•methanesulfonic acid salt

To 5ml of N,N-dimethylformamide, 133mg of (2RS)-1-O-hexadecyl-2-O-methyl-3-O-[7-(methanesulfonyloxy)heptyl]glycerol (prepared in Reference Example 16) and 2ml of 30% aqueous solution of trimethylamine were added and the mixture was stirred for 2 hours at 60°C. The reaction mixture was concentrated under reduced pressure and the residue was purified by column chromatography on silica gel (eluent; chloroform : mathanol = 10 : 1→ chloroform : methanol : water = 65 : 35 : 2) to give 92mg of the title compound having the following physical characteristics:

Melting point : 35 - 38°C;

TLC(ethyl acetate : acetic acid : water = 3 : 1 : 1) : Rf = 0.43;

NMR : δ = 3.60 - 3.30 (14H, m), 3.34 (9H, s),
2.74 (3H, s), 2.00 - 1.20 (38H, m),
0.87 (3H, m);

IR : ν = 3450, 2920, 2850, 1470, 1120 $cm^{-1}$;

Mass : m/z = 471, 456, 440.

By the same procedure as described in Example 5, the following compounds were obtained by using the various glycerol compounds prepared in Reference Example 16 instead of 2-O-methylglycerol compound used in Example 5.

(a) (2RS)-1-O-hexadecyl-2-O-ethyl-3-O-[7-(trimethylammonio) heptyl]glycerol·methanesulfonic acid salt

Starting material : (2RS)-1-O-hexadecyl-2-O-ethyl-3-O-[7-(methanesulfonyloxy)heptyl]glycerol prepared in Reference Example 16 (a);

Melting point : 38 - 45°C;

TLC(ethyl acetate : acetic acid : water = 3 : 1 : 1) : Rf = 0.43;

NMR : δ = 3.62 (2H, q), 3.70 - 3.23 (11H, m),
3.34 (9H, s), 2.74 (3H, s),
1.96 - 1.18 (38H, m), 1.18 (3H, t),
0.86 (3H, m);

IR : ν = 3450, 2930, 2850, 1470, 1120 $cm^{-1}$;

Mass : m/z = 485, 456, 440.

(b) (2RS)-1-O-hexadecyl-2-O-n-propyl-3-O-[7-(trimethylammonio) heptyl] glycerol·methanesulfonic acid salt

Starting material : (2RS)-1-O-hexadecyl-2-O-n-propyl-3-O-[7-(methanesulfonyloxy)heptyl]glycerol prepared in Reference Example 16 (b);

TLC(ethyl acetate : acetic acid : water = 3 : 1 : 1) : Rf = 0.45;

NMR : δ = 3.60 - 3.30 (13H, m), 3.34 (9H, s), 2.74 (3H, s), 1.95 - 1.12 (40H, m), 0.90 (3H, t), 0.87 (3H, m);

IR : ν = 3450, 2920, 2850, 1460, 1110 $cm^{-1}$;

Mass : m/z = 499, 456, 440.

(c) (2RS)-1-O-hexadecyl-2-O-hexadecyl-3-O-[4-(trimethylammonio)butyl]glycerol·methanesulfonic acid salt

Starting material : (2RS)-1-O-hexadecyl-2-O-hexadecyl-3-O-[4-(methanesulfonyloxy)butyl]glycerol prepared in Reference Example 16 (d);

TLC(ethyl acetate : acetic acid : water = 3 : 1 : 1) : Rf = 0.68;

NMR : δ = 3.70 - 3.30 (13H, m), 3.36 (9H, s), 2.77 (3H, s), 2.00 - 1.44 (8H, m), 1.26 (52H, m), 0.88 (6H, m);

IR : ν = 2920, 2850, 1470, 1120 $cm^{-1}$;

Mass : m/z = 639, 414, 398.

Example 6

(2RS)-1-O-hexadecyl-2-O-allyl-3-O-[4-(dimethylamino)butyl]glycerol

By the same procedure as described in Example 1, the title compound having the following physical characteristics was obtained by using 2-O-allylglycerol compound prepared in

Reference Example 16 (c) instead of 2-dehydroxy-2-n-propylglycerol compound used in Example 1.

Starting material : (2RS)-1-O-hexadecyl-2-O-allyl-3-O-[4-(methanesulfonyloxy)butyl]glycerol prepared in Reference Example 16 (c);

TLC(ethyl acetate : acetic acid : water = 3 : 1 : 1) : Rf = 0.42;

NMR : δ = 6.17 - 5.47 (1H, m), 5.47 - 4.93 (2H, m), 4.28 - 4.00 (2H, m), 3.83 - 3.00 (8H, m), 2.53 - 2.00 (2H, m), 2.20 (6H, s), 2.00 - 0.67 (35H, m);

IR : ν = 2925, 2855, 1465, 1380, 1270, 1120, 920 $cm^{-1}$;

Mass : m/z = 4.55 ($M^+$), 454, 440, 414, 398, 308, 230, 200, 148, 130, 117, 116, 100.

Example 7

(2RS)-1-O-hexadecyl-2-O-allyl-3-O-[4-(trimethylammonio)butyl] glycerol·iodide

By the same procedure as described in Example 2, the title compound having the following physical characteristics was obtained by using 2-O-allylglycerol compound (prepared in Example 6) instead of 2-dehydroxy-2-n-propyl-glycerol compound used in Example 2.

Starting material : (2RS)-1-O-hexadecyl-2-O-allyl-3-O-[4-(dimethylamino)butyl]glycerol prepared in Example 6;

Melting point : 53 - 59°C;

TLC(ethyl acetate : acetic acid : water = 3 : 1 : 1) : Rf = 0.34;

NMR : $\delta$ = 6.00 - 5.80 (1H, m), 5.37 - 5.12 (2H, m), 4.20 - 4.10 (2H, m), 3.75 - 3.28 (11H, m), 3.44 (9H, s), 2.00 - 1.48 (6H, m), 1.44 - 1.15 (26H, m), 0.88 (3H, t);

IR(KBr method) : $\nu$ = 2920, 2850, 1480, 1465, 1120, 975, 915, 705 $cm^{-1}$;

Mass : m/z = 455, 454, 413, 398, 308, 230, 200, 183, 158, 142, 117, 116, 100.

Reference Example 17

(2RS)-1-O-hexadecyl-2-O-ethyl-3-O-[8-(tetrahydropyran-2-yloxy) octanoyl]glycerol

To 10ml of methylene chloride, 300mg of (2RS)-1-O-hexadecyl-2-O-ethylglycerol (prepared in Reference Example 10), 276.6mg of 8-(tetrahydropyran-2-yloxy)octanoic acid, 233.5mg of dicyclohexylcarbodiimide and 4mg of dimethylaminopyridine were added, and the mixture was stirred overnight at ambient. The reaction mixture was filtered and the filtrate was concentrated under reduced pressure to give 497mg of the title compound having the following physical characteristic:

TLC(n-hexane : ethyl acetate = 5 : 1 ) : Rf = 0.50.

Reference Example 18

(2RS)-1-O-hexadecyl-2-O-ethyl-3-O-(8-hydroxyoctanoyl)glycerol

To 510mg of tetrahydropyran-2-yloxy compound (prepared in Reference Example 17), 10ml of the mixture of acetic acid-tetrahydrofuran-water (3 : 1 : 1) was added and after the mixture was stirred for 5 hours at 60°C, extracted with ethyl

acetate and the extract was washed with water and a saturated aqueous solution of sodium chloride successively, dried over sodium anhydroussulfate and concentrated under reduced pressure. The residue was purified by column chromatography on silica gel (eluent ; ethyl acetate : n-hexane = 1 : 2) to give 414mg of the title compound having the following physical characteristics:

Starting material : (2RS)-1-O-hexadecyl-2-O-ethyl-3-O-[8-(tetrahydropyran-2-yloxy)octanoyl]glycerol prepared in Reference Example 17.

TLC(chloroform : acetone = 10 : 1) : Rf = 0.54 ;

NMR : $\delta$ = 4.3 - 4.0 (2H, m), 4.0 - 3.2 (9H, m), 2.6 - 2.0 (3H, m), 2.0 - 0.6 (44H, m);

IR : $\nu$ = 3450, 2920, 2860, 1740 $cm^{-1}$;

Mass : m/z = 486 ($M^+$), 468, 456, 411.

Reference Example 19

(2RS)-1-O-hexadecyl-2-O-ethyl-3-O-[8-(methanesulfonyloxy)octanoyl] glycerol

By the same procedure as described in Reference Example 16, the title compound having the following physical characteristic was obtained by using 2-O-ethyl-3-O-(8-hydroxyoctanoyl)glycerol compound (prepared in Reference Example 18) instead of 2-O-methyl-3-O-(7-hydroxyheptyl)glycerol compound used in Reference Example 16.

Starting material : (2RS)-1-O-hexadecyl-2-O-ethyl-3-O-(8-hydroxyoctanoyl)glycerol prepared in Reference Example 18;

TLC(chloroform : acetone = 10 : 1) : Rf = 0.76.

Example 8

(2RS)-1-O-hexadecyl-2-O-ethyl-3-O-[8-(trimethylammonio)octanoyl] glycerol·methanesulfonic acid salt

By the same procedure as described in Example 5, the title compound having the following physical characteristics was obtained by using 2-O-ethyl-3-O-[8-(methanesulfonyloxy)octanoyl] glycerol compound (prepared in Reference Example 19) instead of 2-O-methyl-3-O-[7-(methanesulfonyloxy)heptyl]glycerol compound used in Example 5.

Starting material : (2RS)-1-O-hexadecyl-2-O-ethyl-3-O-[8-(methanesulfonyloxy)octanoyl]glycerol prepared in Reference Example 19;

TLC(ethyl acetate : acetic acid : water = 3 : 1 : 1) : Rf = 0.66;

NMR : δ = 4.30 - 4.00 (2H, m), 3.62 (2H, q),
3.90 - 3.56 (1H, m), 3.55 - 3.30 (6H, m),
3.34 (9H, m), 2.76 (3H, s), 2.32 (2H, t),
2.00 - 1.20 (38H, m), 1.19 (3H, t),
0.88 (3H, m);

IR : ν = 3450, 2920, 2860, 1730, 1470, 1120 $cm^{-1}$;

Mass : m/z = 513, 468.

Reference Example 20

(2RS)-1-O-hexadecyl-2-O-ethyl-3-O-[6-(tetrahydropyran-2-yloxy) hexyl]glycerol

By the same procedure as described in Reference Example 12, the title compound having the following physical

characteristics was obtained by using 2-[6-(p-toluenesulfonyloxy)hexyloxy]tetrahydropyran instead of 2-[7-(p-toluenesulfonyloxy)heptyloxy]tetrahydropyran used in Reference Example 12.

Starting material : (2RS)-1-O-hexadecyl-2-O-ethylglycerol prepared in Reference Example 10;

TLC(n-hexane : ethyl acetate = 5 : 1) : Rf = 0.56;

NMR : $\delta$ = 4.6 (1H, m), 4.0 - 3.3 (15H, m), 2.0 - 1.0 (45H, m), 0.9 (3H, m);

Mass : m/z = 444, 397.

Reference Example 21

(2RS)-1-O-hexadecyl-2-O-ethyl-3-O-(6-hydroxyhexyl)glycerol

By the same procedure as described in Reference Example 15, the title compound having the following physical characteristic was obtained.

Starting material : (2RS)-1-O-hexadecyl-2-O-ethyl-3-O-[6-(tetrahydropyran-2-yloxy)hexyl]glycerol prepared in Reference Example 20;

TLC(n-hexane : ethyl acetate = 5 : 1 ) : Rf = 0.20.

Reference Example 22

(2RS)-1-O-hexadecyl-2-O-ethyl-3-O-[6-(methanesulfonyloxy)hexyl]glycerol

By the same procedure as described in Reference Example 16, the title compound having the following physical characteristic was obtained.

Starting material : (2RS)-1-O-hexadecyl-2-O-ethyl-3-O-

(6-hydroxyhexyl)glycerol prepared in Reference Example 21;

TLC(chloroform : methanol = 10 : 1) : Rf = 0.73.

Example 9

(2RS)-1-O-hexadecyl-2-O-ethyl-3-O-[6-(di-n-butylamino)hexyl] glycerol

By the same procedure as described in Example 1, the title compound having the following physical characteristics was obtained by using di-n-butylamine instead of dimethylamine used in Example 1.

Starting material : (2RS)-1-O-hexadecyl-2-O-ethyl-3-O-[6-(methanesulfonyloxy)hexyl]glycerol prepared in Reference Example 22;

TLC(ethyl acetate : acetic acid : water = 3 : 1 : 1) : Rf = 0.55;

NMR : δ = 3.65 (2H, q), 3.6 - 3.3 (9H, m),
2.6 - 2.2 (6H, m), 1.8 - 1.1 (47H, m),
1.1 - 0.8 (9H, m);

Mass : m/z = 555 ($M^+$), 526, 512.

Example 10

(2RS)-1-O-hexadecyl-2-O-ethyl-3-O-[6-(N,N-di-n-butyl-N-methylammonio)hexyl]glycerol·iodide

By the same procedure as described in Example 2, the title compound having the following physical characteristics was obtained.

Starting material : (2RS)-1-O-hexadecyl-2-O-ethyl-3-O-[6-(di-n-butylamino)hexyl]glycerol prepared

in Example 9;

TLC(ethyl acetate : acetic acid : water = 3 : 1 : 1) : Rf = 0.66;

NMR : $\delta$ = 3.73 - 3.38 (17H, m), 3.32 (2H, s),

1.20 (3H, t, J = 8.0),

1.02 (6H, t, J = 6.0);

IR : $\nu$ = 1940, 1850, 1460, 1110 $cm^{-1}$

Mass : m/z = 554, 512, 470.

Example 11

Five grams of (2RS)-1-O-hexadecyl-2-dehydroxy-2-n-propyl-3-O-[4-(trimethylammonio)butyl]glycerol iodide, 200mg of cellulose calcium gluconate (disintegrator), 100mg of magnesium stearate (lubricating agent) and 9.7g of crystal cellulose were mixed and punched out in a conventional method to obtain tablets containing 50mg of the active ingredient in one tablet.

CLAIMS:

1) A glycerol derivatives of the general formula:

$$\begin{array}{ll} 1 & \ulcorner O\text{-}R^1 \\ 2 & \vdash A\text{-}R^2 \\ 3 & \llcorner B\text{-}R^3\text{-}R^4 \end{array} \qquad (I)$$

[wherein $R^1$ represents an alkyl group of 6 to 22 carbon atoms or an alkyl group of 2 to 5 carbon atoms substituted by a phenyl group, A represents a single bond or an oxygene atom, $R^2$ represents an alkyl group of 1 to 20 carbon atoms, an alkenyl group of 2 to 20 carbon atoms when A represents a single bond or an alkenyl group of 3 to 20 carbon atoms when A represents an oxygene atom, B represents an oxygene atom or a carbonyloxy group (i.e. -O-CO- group; with the proviso that in which a carbonyl group is attached to $R^3$ and an oxa-group is attached to a carbon atom at the third position of the glycerol skeleton), $R^3$ represents an alkylene group of 1 to 12 carbon atoms and $R^4$ represents a group of the formula : $-NHCOR^5$, $-N(R^6)_2$ or $-\overset{\oplus}{N}(R^6)_3 \cdot \overset{\ominus}{Y}$ (in which $R^5$ represents an alkyl group of 1 to 6 carbon atoms or a phenyl group, $R^6$ represents an alkyl group of 1 to 6 carbon atoms and $\overset{\ominus}{Y}$ represents an anion of an acid; with the proviso that when plural $R^6$ are attached to a nitrogen atom, they may be same or different to each other)], and

acid addition salt thereof.

2) A compound according to claim 1, wherein $R^1$ is an alkyl group of 14 to 18 carbon atoms.

3) A compound according to claim 1 or 2, wherein $R^1$ is a hexadecyl group.

4) A compound according to any one of claim 1 to 3, wherein B represents an oxygen atom.

5) A compound according to any one of claim 1 to 3, wherein B represents a carbonyloxy group.

6) A compound according to any one of claim 1 to 5, wherein A represents a single bond and $R^2$ represents an alkyl group of 1 to 6 carbon atoms or an alkenyl group of 2 to 6 carbon atoms.

7) A compound according to any one of claim 1 to 5, wherein A represents an oxygen atom and $R^2$ represents an alkyl group of 1 to 6 carbon atoms or an alkenyl group of 3 to 6 carbon atoms.

8) A compound according to any one of claim 1 to 7, wherein $R^3$ represents an alkylene group of 3 to 8 carbon atoms.

9) A compound according to any one of claim 1 to 8, wherein $R^4$ represents a group of the formula : $-N(R^6)_2$ or $-\overset{\oplus}{N}(R^6)_3 \cdot \overset{\ominus}{Y}$ (in which $R^6$ represents an alkyl group of 1 to 6 carbon atoms and $\overset{\ominus}{Y}$ represents an anion of an acid).

10) A compound according to claim 1, which is:

(2RS)-1-O-hexadecyl-2-dehydroxy-2-n-propyl-3-O-[4-(dimethylamino) butyl]glycerol,

(2RS)-1-O-hexadecyl-2-dehydroxy-2-iso-propyl-3-O-

[4-(dimethylamino)butyl] glycerol,

(2RS)-1-O-hexadecyl-2-dehydroxy-2-n-pentyl-3-O-[4-(dimethylamino)butyl] glycerol,

(2RS)-1-O-hexadecyl-2-dehydroxy-2-iso-pentyl-3-O-[4-(dimethylamino)butyl] glycerol,

(2RS)-1-O-hexadecyl-2-dehydroxy-2-n-decyl-3-O-[4-(dimethylamino)butyl] glycerol,

(2RS)-1-O-hexadecyl-2-dehydroxy-2-n-propyl-3-O-[4-(trimethylammonio)butyl]glycerol·iodide,

(2RS)-1-O-hexadecyl-2-dehydroxy-2-iso-propyl-3-O-[4-(trimethylammonio)butyl]glycerol·iodide,

(2RS)-1-O-hexadecyl-2-dehydroxy-2-n-propyl-3-O-[4-(N,N-dimethyl-N-ethylammonio)butyl]glycerol·iodide,

(2RS)-1-O-hexadecyl-2-dehydroxy-2-n-pentyl-3-O-[4-(trimethylammonio)butyl]glycerol·iodide,

(2RS)-1-O-hexadecyl-2-dehydroxy-2-iso-pentyl-3-O-[4-(trimethylammonio)butyl]glycerol·iodide,

(2RS)-1-O-hexadecyl-2-dehydroxy-2-n-decyl-3-O-[4-(trimethylammonio)butyl]glycerol·iodide,

(2RS)-1-O-hexadecyl-2-O-ethyl-3-O-[4-(dimethylamino)butyl] glycerol,

(2RS)-1-O-hexadecyl-2-O-ethyl-3-O-[4-(trimethylammonio)butyl] glycerol·iodide,

(2RS)-1-O-hexadecyl-2-O-allyl-3-O-[4-(dimethylamino)butyl] glycerol,

(2RS)-1-O-hexadecyl-2-O-allyl-3-O-[4-(trimethylammonio)butyl] glycerol·iodide,

(2RS)-1-O-hexadecyl-2-O-ethyl-3-O-[6-(N,N-di-n-butyl-N-methylammonio)hexyl]glycerol·iodide,

(2RS)-1-O-hexadecyl-2-O-methyl-3-O-[7-(trimethylammonio)heptyl]glycerol·methanesulfonic acid salt,

(2RS)-1-O-hexadecyl-2-O-ethyl-3-O-[7-(trimethylammonio)heptyl]glycerol·methanesulfonic acid salt,

(2RS)-1-O-hexadecyl-2-O-n-propyl-3-O-[7-(trimethylammonio)heptyl]glycerol·methanesulfonic acid salt,

(2RS)-1-O-hexadecyl-2-O-n-hexadecyl-3-O-[4-(trimethylammonio)butyl]glycerol·methanesulfonic acid salt,

or

(2RS)-1-O-hexadecyl-2-O-ethyl-3-O-[8-(trimethylammonio)octanoyl]glycerol·methanesulfonic acid salt.

11) A process for the preparation of compounds of the general formula:

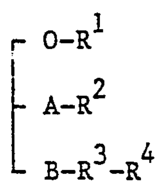

(I)

(wherein $R^1$ represents an alkyl group of 6 to 22 carbon atoms or an alkyl group of 2 to 5 carbon atoms substituted by a phenyl group, A represents a single bond or an oxygen atom, $R^2$ represents an alkyl group of 1 to 20 carbon atoms, an alkenyl group of 2 to 20 carbon atoms when A represents a single bond or an alkenyl group of 3 to 20 carbon atoms when A represents an oxygen atom, B represents an oxygen atom or a carbonyloxy group (i.e. -O-CO- group; with the proviso that in which a carbonyl group is attached to $R^3$ and an oxa-group is attached to a

carbon atom at the third position of the glycerol skeleton), $R^3$ represents an alkylene group of 1 to 12 carbon atoms and $R^4$ represents a group of the formula : $-NHCOR^5$, $-N(R^6)_2$ or $-\overset{\oplus}{N}(R^6)_3 \cdot \overset{\ominus}{Y}$ (in which $R^5$ represents an alkyl group of 1 to 6 carbon atoms or a phenyl group, $R^6$ represents an alkyl group of 1 to 6 carbon atoms and $\overset{\ominus}{Y}$ represents an anion of an acid; with the proviso that when plural $R^6$ are attached to a nitrogen atom, they may be same or different to each other) .

(i) which comprises the reaction of a compound of the general formula:

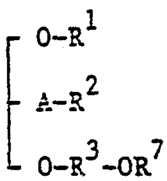

(IIa)

or

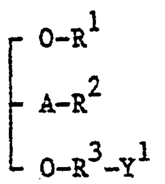

(IIIa)

(wherein $R^7$ represents an alkylsulfonyl group or a substituted or unsubstituted arylsulfonyl group, $Y^1$ represents a halogen atom and the other symbols are as hereinbefore defined), with a compound of the general formula:

$$NH(R^6)_2 \text{ or } N(R^6)_3$$

(wherein $R^6$ represents an alkyl group of 1 to 6 carbon atoms),

(ii) which comprises the esterification of a compound of the general formula:

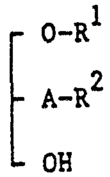

(IV)

(wherein all the symbols are as hereinbefore defined), with a compound of the general formula:

$HOOC-R^3-(R^6)_2$ (IV-1)

(wherein all the symbols are as hereinbefore defined),

(iii) which comprises the reaction of a compound of the general formula:

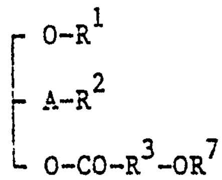

(IIb)

or

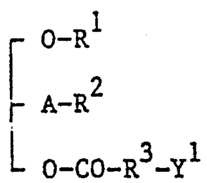

(IIIb)

(wherein all the symbols are as hereinbefore defined), with a compound of the general formula:

$N(R^6)_3$

(wherein $R^6$ is as hereinbefore defined),

(iv) which comprises the acylation of a compound of the general formula:

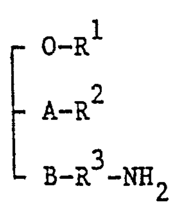

(Ie')

(wherein all the symbols are as hereinbefore defined)

or

(v) which comprises the reaction of a compound of the general formula:

$$\left[\begin{array}{l} O-R^1 \\ A-R^2 \\ B-R^3-N(R^6)_2 \end{array}\right. \qquad (Ig)$$

(wherein all the symbols are as hereinbefore defined), with an alkyl halide.

12) A platelet aggregation inhibiting, anti-asthmatic, anti-allergic, anti-inflammatory, hypotensive or anti-tumour pharmaceutical composition which comprises, as active ingredient, an effective amount of at least one compound of the general formula I depicted in claim 1, wherein the various symbols are as defined in claim 1, or a non-toxic acid addition salt thereof, together with a pharmaceutical carrier or coating.

13) A method of inhibiting platelet aggregation, or for the prevention and treatment of asthma, allergy, inflammation, hypertension and tumour which comprises the oral or parenteral administration of an effective amount of a compound as claimed in claim 1, wherein the various symbols are as defined in claim 1, or a non-toxic acid addition salt thereof.